# EUROPEAN PATENT SPECIFICATION

(11) **EP 1 898 909 B1**
(45) Date of publication and mention of the grant of the patent: **19.10.2016**
(21) Application number: 06763888.2
(22) Date of filing: 26.06.2006
(51) Int. Cl.: A61K 31/4353, A61P 31/04, C07D 215/227, C07D 215/36

(54) **QUINOLINE DERIVATIVES AS ANTIBACTERIAL AGENTS**
CHINOLINDERIVATE ALS ANTIBAKTERIELLE WIRKSTOFFE
DÉRIVÉS DE LA QUINOLÉINE COMME AGENTS ANTIBACTÉRIENS

(30) Priority: 28.06.2005 EP 05105755
(43) Date of publication of application: 19.03.2008
(73) Proprietor: Janssen Pharmaceutica NV, 2340 Beerse (BE)
(72) Inventor: KOUL, Anil, B-2340 Beerse (BE); ANDRIES, Koenraad, Jozef, Lodewijk, B-2340 Beerse (BE); GUILLEMONT, Jérome, Emile, Georges, 27106 Val de Reuil Cedex (FR); MOTTE, Magali, Madeleine, Simone, 27106 Val de Reuil Cedex (FR); LANÇOIS, David, Francis, Alain, 27106 Val de Reuil Cedex (FR)
(74) Representative: Vervoort, Liesbeth
(86) International application number: PCT/EP2006/063552
(87) International publication number: WO 2007/000434

(56) References cited:
- WO-A-2004/011436
- WO-A-2005/117875
- WO-A1-2006/131519
- HELWIG, BURGHARD: "Moderne Arzneimittel" 1980, WISSENSCHAFTLICHE VERLAGSGESELLSCHAFT , STUTTGART , XP002359316 page 395
- ANDRIES, KOEN ET AL.: SCIENCE, vol. 307, 14 January 2005 (2005-01-14), pages 223-227, XP002358962 cited in the application
- DESAI P K ET AL: "QUINOLINE DERIVATIVES AS ANTITUBERCULAR/ANTIBACTERIAL AGENTS" INDIAN JOURNAL OF CHEMISTRY, JODHPUR, IN, vol. 35B, no. 8, August 1996 (1996-08), pages 871-873, XP000944820
- ANDRIES, K.: 'Supporting Online Material', [Online] Supporting material to Andries: science 307, 223 (2005), XP2358962 Retrieved from the Internet: <URL:www.sciencemag.org/content/suppl/2005/ 01/18/1106753.DC1/Andries.SOM.pdf> [retrieved on 2013-01-16]
- STEPHEN M BERGE ET AL: 'Pharmaceutical salts', [Online] vol. 66, no. 1, 01 January 1977, pages 1 - 19, XP002675560 DOI: 10.1002/JPS.2600660104 ISSN: 0022-3549 JOURNAL OF PHARMACEUTICAL SCIENCES, AMERICAN PHARMACEUTICAL ASSOCIATION, WASHINGTON, US Retrieved from the Internet: <URL:http://onlinelibrary.wiley.com/doi/10. 1002/jps.2600660104/abstract> [retrieved on 2006-09-18]

## Description

The present invention relates to the use of quinoline derivatives for the manufacture of a medicament for the treatment of a bacterial infection.

Resistance to first-line antibiotic agents is an emerging problem. Some important examples include penicillin-resistant *Streptococcus pneumoniae,* vancomycin-resistant enterococci, methicillin-resistant *Staphylococcus aureus,* multi-resistant salmonellae.

The consequences of resistance to antibiotic agents are severe. Infections caused by resistant microbes fail to respond to treatment, resulting in prolonged illness and greater risk of death. Treatment failures also lead to longer periods of infectivity, which increase the numbers of infected people moving in the community and thus exposing the general population to the risk of contracting a resistant strain infection.

Hospitals are a critical component of the antimicrobial resistance problem worldwide. The combination of highly susceptible patients, intensive and prolonged antimicrobial use, and cross-infection has resulted in infections with highly resistant bacterial pathogens.

Self-medication with antimicrobials is another major factor contributing to resistance. Self-medicated antimicrobials may be unnecessary, are often inadequately dosed, or may not contain adequate amounts of active drug.

Patient compliance with recommended treatment is another major problem. Patients forget to take medication, interrupt their treatment when they begin to feel better, or may be unable to afford a full course, thereby creating an ideal environment for microbes to adapt rather than be killed.

Because of the emerging resistance to multiple antibiotics, physicians are confronted with infections for which there is no effective therapy. The morbidity, mortality, and financial costs of such infections impose an increasing burden for health care systems worldwide.

Therefore, there is a high need for new compounds to treat bacterial infections, especially for the treatment of infections caused by resistant strains.

WO 2004/011436 discloses substituted quinoline derivatives having activity against Mycobacteria, in particular against *Mycobacterium tuberculosis*. One particular compound of these substituted quinoline derivatives is described in Science (2005), 307, 223-227.

It has now been found that quinoline derivatives described in WO 2004/011436 also show activity against other bacteria than Mycobacteria.

Therefore, the present invention relates to the use of a compound for the manufacture of a medicament for the treatment of a bacterial infection caused by Staphylococci, Enterococci or Streptococci, said compound being a compound of formula (Ia) or (Ib) 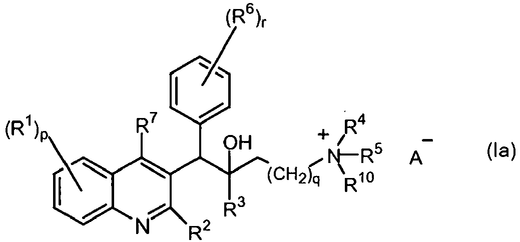 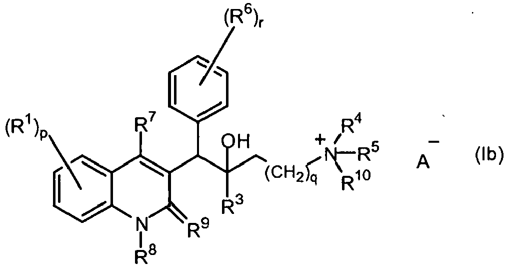 a *N*-oxide thereof, a tautomeric form thereof or a stereochemically isomeric form thereof wherein
- A-: is a pharmaceutically acceptable counter ion;
- R¹: is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
- p: is an integer equal to 1, 2, 3 or 4 ;
- R²: is hydrogen, hydroxy, mercapto, alkyloxy, alkyloxyalkyloxy, alkylthio, mono or di(alkyl)amino or a radical of formula 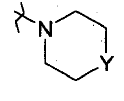 wherein Y is CH₂, O, S, NH or *N*-alkyl ;
- R³: is alkyl, Ar, Ar-alkyl, Het or Het-alkyl;
- q: is an integer equal to zero, 1, 2, 3 or 4 ;
- R⁴ and R⁵: each independently are hydrogen, alkyl or benzyl;
- R⁴ and R⁵: together and including the N to which they are attached may form a radical selected from the group of pyrrolidinyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, pyridazinyl, pyrimidinyl, pyrazinyl, triazinyl, morpholinyl and thiomorpholinyl, each of said rings may optionally be substituted with alkyl, halo, haloalkyl, hydroxy, alkyloxy, amino, mono- or dialkylamino, alkylthio, alkyloxyalkyl, alkylthioalkyl, Ar-alkyl or pyrimidinyl;
- R⁶: is hydrogen, halo, haloalkyl, hydroxy, Ar, alkyl, alkyloxy, alkylthio, alkyloxyalkyl, alkylthioalkyl, Ar-alkyl or di(Ar)alkyl ; or
two vicinal R⁶ radicals may be taken together to form a bivalent radical of formula -CH=CH-CH=CH- ;
- r: is an integer equal to 1, 2, 3, 4 or 5 ;
- R⁷: is hydrogen, alkyl, Ar or Het ;
- R⁸: is hydrogen or alkyl,
- R⁹: is oxo ; or
- R⁸ and R⁹: together form the radical -CH=CH-N=;
- R¹⁰: is alkyl, alkylcarbonyl, Ar, Ar-alkyl, Ar-carbonyl, Het¹-alkyl or Het¹-carbonyl;
- alkyl: is a straight or branched saturated hydrocarbon radical having from 1 to 6 carbon atoms ; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with hydroxy, alkyloxy or oxo ;
- Ar: is a homocycle selected from the group of phenyl, naphthyl, acenaphthyl, tetrahydronaphthyl, each homocycle optionally substituted with 1, 2 or 3 substituents, each substituent independently selected from the group of hydroxy, halo, cyano, nitro, amino, mono- or dialkylamino, alkyl, haloalkyl, alkyloxy, Het haloalkyloxy, carboxyl, alkyloxycarbonyl, aminocarbonyl, morpholinyl and mono- or dialkylaminocarbonyl ; is a monocyclic heterocycle selected from the group of *N*-phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl and benzo[1,3]dioxolyl ; each monocyclic and bicyclic heterocycle may optionally be substituted with 1, 2 or 3 substituents, each substituent independently selected from the group of halo, hydroxy, alkyl, alkyloxy, and Ar-carbonyl;
- Het¹: is a monocyclic heterocycle selected from furanyl or thienyl; or a bicyclic heterocycle selected from benzofuranyl or benzothienyl; each monocyclic and bicyclic heterocycle may optionally be substituted with 1, 2 or 3 substituents, each substituent independently selected from the group of halo, alkyl and Ar;
- halo: is a substituent selected from the group of fluoro, chloro, bromo and iodo; and
- haloalkyl: is a straight or branched saturated hydrocarbon radical having from 1 to 6 carbon atoms or a cyclic saturated hydrocarbon radical having from 3 to 6 carbon atoms or a cyclic saturated hydrocarbon radical having from 3 to 6 carbon atoms attached to a straight or branched saturated hydrocarbon radical having from 1 to 6 carbon atoms; wherein one or more carbon atoms are substituted with one or more halo atoms.

The present invention also concerns a method of treating a bacterial infection in a mammal, in particular a warm-blooded mammal, more in particular a human, comprising administering an effective amount of a compound of the invention to the mammal.

The present invention also concerns a compound of formula (Ia) or (Ib) 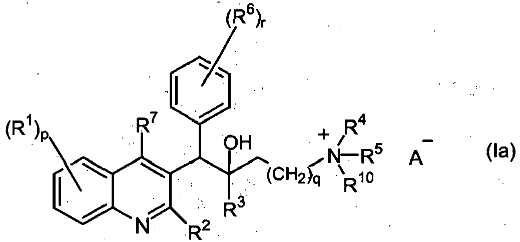 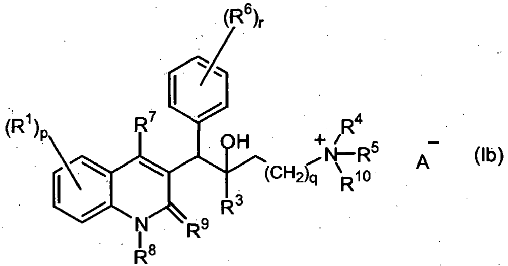 a *N*-oxide thereof, a tautomeric form thereof or a stereochemically isomeric form thereof wherein
A- is a pharmaceutically acceptable counter ion;
R¹ is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
p is an integer equal to 1, 2, 3 or 4 ;
R² is hydrogen, hydroxy, mercapto, alkyloxy, alkyloxyalkyloxy, alkylthio, mono or di(alkyl)amino or a radical of formula 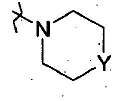 wherein Y is
R³ CH₂, O, S, NH or *N*-alkyl ; is alkyl, Ar, Ar-alkyl, Het or Het-alkyl;
q is an integer equal to zero, 1, 2, 3 or 4 ;
R⁴ and R⁵ each independently are hydrogen, alkyl or benzyl;
R⁴ and R⁵ together and including the N to which they are attached may form a radical selected from the group of pyrrolidinyl, 2-pyrrolinyl, 3-pyrrolinyl, pyrrolyl, imidazolidinyl, pyrazolidinyl, 2-imidazolinyl, 2-pyrazolinyl, imidazolyl pyrazolyl, triazolyl, piperidinyl, pyridinyl, piperazinyl, pyridazinyl, pyrimidinyl, pyrazinyl, triazinyl, morpholinyl and thiomorpholinyl, each of said rings may optionally be substituted with alkyl, halo, haloalkyl, hydroxy, alkyloxy, amino, mono- or dialkylamino, alkylthio, alkyloxyalkyl, alkylthioalkyl, Ar-alkyl or pyrimidinyl;
R⁶ is hydrogen, halo, haloalkyl, hydroxy, Ar, alkyl, alkyloxy, alkylthio, alkyloxyalkyl, alkylthioalkyl, Ar-alkyl or di(Ar)alkyl ; or two vicinal R⁶ radicals may be taken together to form a bivalent radical of formula

-CH=CH-CH=CH- ;

r is an integer equal to 1, 2, 3, 4 or 5 ;
R⁷ is hydrogen, alkyl, Ar or Het ;
R^{B} is hydrogen or alkyl ;
R⁹ is oxo; or
R⁸ and R⁹ together form the radical -CH=CH-N=;
R¹⁰ is alkyl, alkylcarbonyl, Ar, Ar-alkyl, Ar-carbonyl, Het¹-alkyl or Het¹-carbonyl;
alkyl is a straight or branched saturated hydrocarbon radical having from 1 to 6 carbon atoms; or is a cyclic saturated hydrocarbon radical having from 3 to 6 carbon atoms; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with hydroxy, alkyloxy or oxo ;
Ar is a homocycle selected from the group of phenyl, naphthyl, acenaphthyl, tetrahydronaphthyl, each homocycle optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl ;
Het is a monocyclic heterocycle selected from the group of *N*-phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl and benzo[1,3]dioxolyl; each monocyclic and bicyclic heterocycle may optionally be substituted with 1, 2 or 3 substituents, each substituent independently selected from the group of halo, hydroxy, alkyl, alkyloxy, and Ar-carbonyl;
Het¹ is a monocyclic heterocycle selected from furanyl or thienyl; or a bicyclic heterocycle selected from benzofuranyl or benzothienyl; each monocyclic and halo bicyclic heterocycle may optionally be substituted with 1, 2 or 3 substituents, each substituent independently selected from the group of halo, alkyl and Ar; is a substituent selected from the group of fluoro, chloro, bromo and iodo; and
haloalkyl is a straight or branched saturated hydrocarbon radical having from 1 to 6 carbon atoms or a cyclic saturated hydrocarbon radical having from 3 to 6 carbon atoms or a cyclic saturated hydrocarbon radical having from 3 to 6 carbon atoms attached to a straight or branched saturated hydrocarbon radical having from 1 to 6 carbon atoms; wherein one or more carbon atoms are substituted with one or more halo atoms; provided that when R¹⁰ is alkyl or benzyl, then R⁴ and R⁵ are other than hydrogen; and provided that the compound is other than

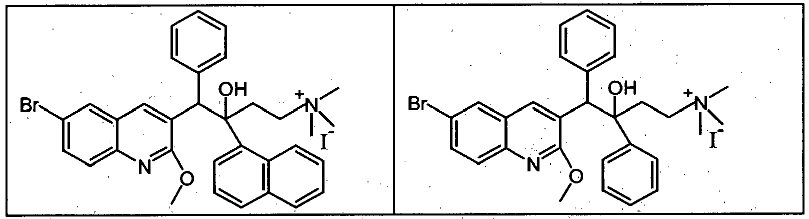

a *N*-oxide thereof, a tautomeric form thereof or a stereochemically isomeric form thereof.

The compounds according to Formula (Ia) and (Ib) are interrelated in that e.g. a compound according to Formula (Ib), with R⁹ equal to oxo is the tautomeric equivalent of a compound according to Formula (Ia) with R² equal to hydroxy (keto-enol tautomerism).

In the framework of this application, alkyl is a straight or branched saturated hydrocarbon radical having from 1 to 6 carbon atoms ; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with hydroxy, alkyloxy or oxo.

Preferably, alkyl is methyl, ethyl or cyclohexylmethyl, more preferably methyl or ethyl. An interesting embodiment of alkyl in all definitions used hereinbefore or hereinafter is C₁₋₆alkyl which represents a straight or branched saturated hydrocarbon radical having from 1 to 6 carbon atoms such as for example methyl, ethyl, propyl, 2-methyl-ethyl, pentyl, hexyl. A preferred subgroup of C₁₋₆alkyl is C₁₋₄alkyl which represents a straight or branched saturated hydrocarbon radical having from 1 to 4 carbon atoms such as for example methyl, ethyl, propyl, 2-methyl-ethyl.

In the framework of this application, Ar is a homocycle selected from the group of phenyl, naphthyl, acenaphthyl, tetrahydronaphthyl, each optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl. Preferably, Ar is naphthyl or phenyl, each optionally substituted with 1 or 2 halo substituents.

In the framework of this application, Het is a monocyclic heterocycle selected from the group of *N*-phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl and benzo[1,3]dioxolyl ; each monocyclic and bicyclic heterocycle may optionally be substituted with 1,2 or 3 substituents, each substituent independently selected from the group of halo, hydroxy, alkyl, alkyloxy and Ar-carbonyl. Preferably, Het is thienyl.

In the framework of this application, halo is a substituent selected from the group of fluoro, chloro, bromo and iodo and haloalkyl is a straight or branched saturated hydrocarbon radical having from 1 to 6 carbon atoms or a cyclic saturated hydrocarbon radical having from 3 to 6 carbon atoms or a cyclic saturated hydrocarbon radical having from 3 to 6 carbon atoms attached to a straight or branched saturated hydrocarbon radical having from 1 to 6 carbon atoms; wherein one or more carbon atoms are substituted with one or more halo atoms. Preferably, halo is bromo, fluoro or chloro and preferably, haloalkyl is polyhaloC₁₋₆alkyl which is defined as mono- or polyhalosubstituted C₁₋₆alkyl, for example, methyl with one or more fluoro atoms, for example, difluoromethyl or trifluoromethyl, 1;1-difluoro-ethyl. In case more than one halo atom is attached to an alkyl group within the definition of haloalkyl or polyhaloC₁₋₆alkyl, they may be the same or different.

In the definition of Het, it is meant to include all the possible isomeric forms of the heterocycles, for instance, pyrrolyl comprises 1*H*-pyrrolyl and 2*H*-pyrrolyl.

The Ar, Het or Het¹ listed in the definitions of the substituents of the compounds of formula (I) (see for instance R³) as mentioned hereinbefore or hereinafter may be attached to the remainder of the molecule of formula (Ia) or (lb) through any ring carbon or heteroatom as appropriate, if not otherwise specified. Thus, for example, when Het is imidazolyl, it may be 1-imidazolyl, 2-imidazolyl, 4-imidazolyl.

Lines drawn from substituents into ring systems indicate that the bond may be attached to any of the suitable ring atoms.

When two vicinal R⁶ radicals are taken together to form a bivalent radical of formula -CH=CH-CH=CH-, this means that the two vicinal R⁶ radicals form together with the phenyl ring to which they are attached a naphthyl.

Pharmaceutically acceptable counterions (A⁻) include chloride, bromide, iodide, trifluoroacetate, acetate, triflate, sulfate, sulfonate. The counterion of choice can be introduced using ion exchange resins.

The *N*-oxide forms of the present compounds are meant to comprise the compounds of formula (Ia) or (lb) wherein one or several tertiary nitrogen atoms are oxidized to the so-called *N*-oxide.

The compounds of formula (Ia) and (Ib) may be converted to the corresponding *N*-oxide forms following art-known procedures for converting a trivalent nitrogen into its *N*-oxide form. Said *N*-oxidation reaction may generally be carried out by reacting the starting material of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. *t*.butyl hydro-peroxide. Suitable solvents are, for example, water, lower alcohols, e.g. ethanol, hydrocarbons, e.g. toluene, ketones, e.g. 2-butanone, halogenated hydrocarbons, e.g. dichloromethane, and mixtures of such solvents.

It will be appreciated that some of the compounds of formula (I) and their *N*-oxides or tautomeric forms may contain one or more centres of chirality and exist as stereochemically isomeric forms.

Compounds of either formula (Ia) and (Ib) and some of the intermediate compounds invariably have at least two stereogenic centers in their structure which may lead to at least 4 stereochemically different structures.

The term "stereochemically isomeric forms" as used hereinbefore or hereinafter defines all the possible stereoisomeric forms which the compounds of formula (Ia) and (Ib), and their *N*-oxides, addition salts or physiologically functional derivatives may possess. Unless otherwise mentioned or indicated, the chemical designation of compounds denotes the mixture of all possible stereochemically isomeric forms, said mixtures containing all diastereomers and enantiomers of the basic molecular structure. In particular, stereogenic centers may have the R- or S-configuration; substituents on bivalent cyclic (partially) saturated radicals may have either the *cis-* or *trans-*configuration. Compounds encompassing double bonds can have an E (entgegen) or Z (zusammen) -stereochemistry at said double bond. The terms cis, trans, R, S, E and Z are well known to a person skilled in the art.
Stereochemically isomeric forms of the compounds of formula (Ia) and (Ib) are obviously intended to be embraced within the scope of this invention.

Following CAS-nomenclature conventions, when two stereogenic centers of known absolute configuration are present in a molecule, an *R* or *S* descriptor is assigned (based on Cahn-Ingold-Prelog sequence rule) to the lowest-numbered chiral center, the reference center. The configuration of the second stereogenic center is indicated using relative descriptors [*R**,*R**] or [*R**,*S**], where *R** is always specified as the reference center and [*R*,R**] indicates centers with the same chirality and [*R*,S**] indicates centers of unlike chirality. For example, if the lowest-numbered chiral center in the molecule has an *S* configuration and the second center is *R*, the stereo descriptor would be specified as *S-*[*R*,S**]. If "*α*" and "*β*" are used : the position of the highest priority substituent on the asymmetric carbon atom in the ring system having the lowest ring number, is arbitrarily always in the "*α*" position of the mean plane determined by the ring system. The position of the highest priority substituent on the other asymmetric carbon atom in the ring system relative to the position of the highest priority substituent on the reference atom is denominated "*α*", if it is on the same side of the mean plane determined by the ring system, or "*β*", if it is on the other side of the mean plane determined by the ring system.

When a specific stereoisomeric form is indicated, this means that said form is substantially free, *i.e*. associated with less than 50 %, preferably less than 20 %, more preferably less than 10 %, even more preferably less than 5%, further preferably less than 2 % and most preferably less than 1 % of the other isomer(s). Thus, when a compound of formula (I) is for instance specified as (αS, βR), this means that the compound is substantially free of the (αR, βS) isomer.

The compounds of either formula (Ia) and (Ib) may be synthesized in the form of racemic mixtures of enantiomers which can be separated from one another following art-known resolution procedures. The racemic compounds of either formula (Ia) and (Ib) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of either formula (Ia) and (Ib) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The tautomeric forms of the compounds of either formula (Ia) and (Ib) are meant to comprise those compounds of either formula (Ia) and (Ib) wherein e.g. an enol group is converted into a keto group (keto-enol tautomerism).

Derivative compounds (usually called "pro-drugs") of pharmacologically-active compounds may be degraded *in vivo* to yield the compounds. Pro-drugs are usually (but not always) of lower potency at the target receptor than the compounds to which they are degraded. Pro-drugs are particularly useful when the desired compound has chemical or physical properties that make its administration difficult or inefficient. For example, the desired compound may be only poorly soluble, it may be poorly transported across the mucosal epithelium, or it may have an undesirably short plasma half-life. Further discussion on pro-drugs may be found in Stella, V. J. et al., "Prodrugs", Drug Delivery Systems, 1985, pp. 112-176, and Drugs, 1985,29, pp. 455-473.

Pro-drugs forms of pharmacologically-active compounds will generally be the pharmaceutically acceptable acid or base addition salts, the stereochemically isomeric forms, the tautomeric forms and the *N*-oxide forms, having an acid group which is esterified or amidated. Included in such esterified acid groups are groups of the formula-COOR^{x}, where R^{x} is a C₁₋₆alkyl, phenyl, benzyl or one of the following groups :

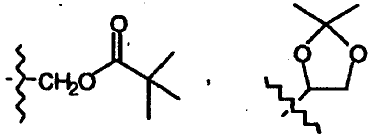

Amidated groups include groups of the formula - CONR^{y}R^{z}, wherein R^{y} is H, C₁₋₆alkyl, phenyl or benzyl and R^{z} is -OH, H, C₁₋₆alkyl, phenyl or benzyl.

Compounds according to the invention having an amino group may be derivatised with a ketone or an aldehyde such as formaldehyde to form a Mannich base. This base will hydrolyze with first order kinetics in aqueous solution.

Whenever used herein, the term "compounds of formula (Ia) or (Ib)" is meant to also include their *N*-oxide forms, their tautomeric forms or their stereochemically isomeric forms. Of special interest are those compounds of formula (Ia) or (Ib) which are stereochemically pure.

A first interesting embodiment of the present invention relates to a compound of Formula (Ia-1) or (Ib-1) 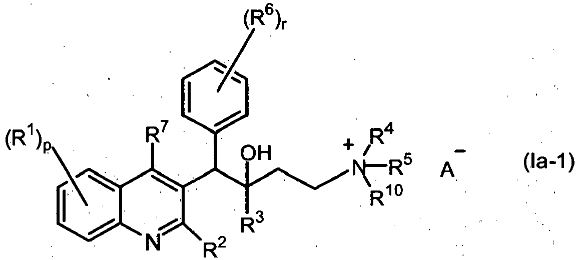 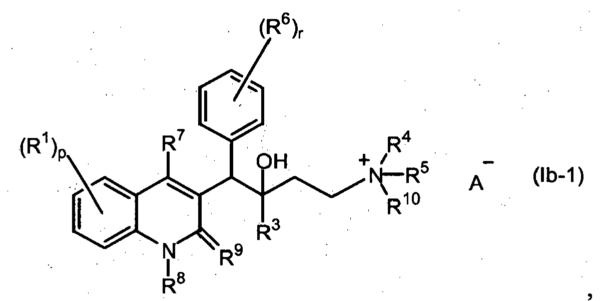 a *N*-oxide thereof, a tautomeric form thereof or a stereochemically isomeric form thereof.

A second interesting embodiment of the present invention relates to a compound of Formula (Ia-2) or (Ib-2) 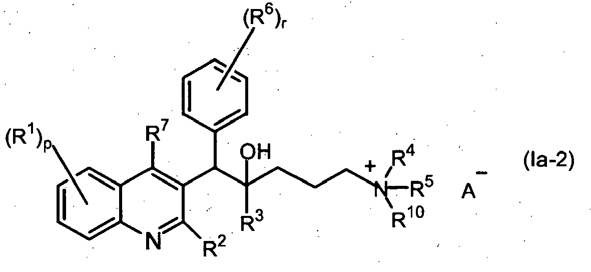 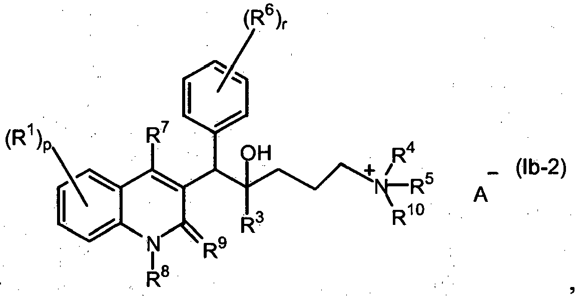 a *N*-oxide thereof, a tautomeric form thereof or a stereochemically isomeric form thereof.

A third interesting embodiment of the present invention relates to a compound of Formula (Ia-3) or (Ib-3) 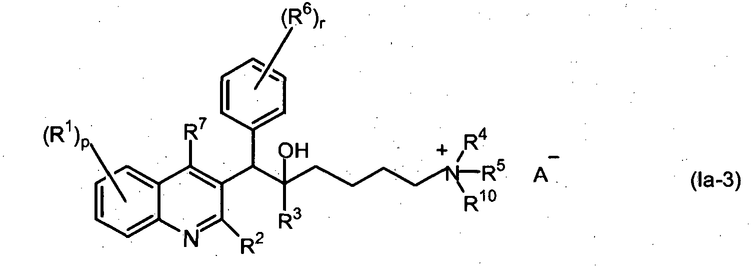 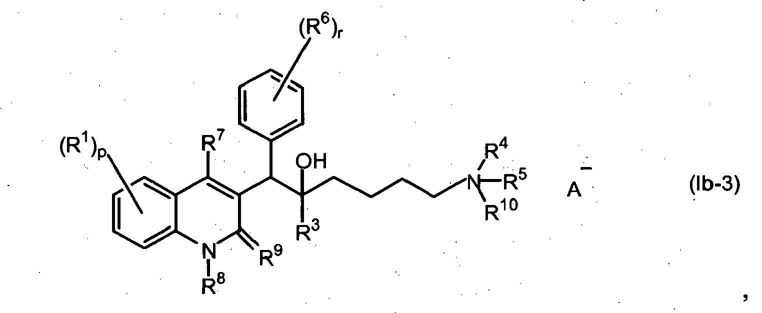 a *N*-oxide thereof, a tautomeric form thereof or a stereochemically isomeric form thereof.

A fourth interesting embodiment relates to a compound of formula (Ia) or (Ib) or any subgroup thereof as mentioned hereinbefore as interesting embodiment wherein the compound has the following formula 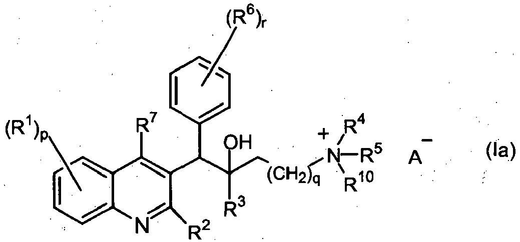 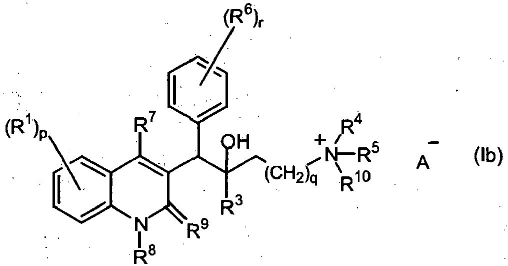 a *N*-oxide thereof, a tautomeric form thereof or a stereochemically isomeric form thereof wherein
- A-: is a pharmaceutically acceptable counter ion;
- R¹: is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
- p: is an integer equal to 1, 2, 3 or 4 ;
- R²: is hydrogen, hydroxy, mercapto, alkyloxy, alkyloxyalkyloxy, alkylthio, mono or di(alkyl)amino or a radical of formula 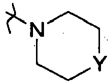 wherein Y is

- R³: CH₂, O, S, NH or *N*-alkyl ; is alkyl, Ar, Ar-alkyl, Het or Het-alkyl;
- q: is an integer equal to zero, 1, 2, 3 or 4 ;
- R⁴ and R⁵: each independently are hydrogen, alkyl or benzyl;
- R⁴ and R⁵: together and including the N to which they are attached may form a radical selected from the group of pyrrolidinyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, pyridazinyl, pyrimidinyl, pyrazinyl, triazinyl, morpholinyl and thiomorpholinyl, each of said rings may optionally be substituted with alkyl, halo, haloalkyl, hydroxy, alkyloxy, amino, mono- or dialkylamino, alkylthio, alkyloxyalkyl, alkylthioalkyl, Ar-alkyl or pyrimidinyl;
- R⁶: is hydrogen, halo, haloalkyl, hydroxy, Ar, alkyl, alkyloxy, alkylthio, alkyloxyalkyl, alkylthioalkyl, Ar-alkyl or di(Ar)alkyl ; or
two vicinal R⁶ radicals may be taken together to form a bivalent radical of formula

-CH=CH-CH=CH- ;

- r: is an integer equal to 1, 2, 3, 4 or 5 ;
- R⁷: is hydrogen, alkyl, Ar or Het ;
- R⁸: is hydrogen or alkyl;
- R⁹: is oxo ;
- R¹⁰: is alkyl, alkylcarbonyl, Ar, Ar-alkyl, Ar-carbonyl, Het¹-alkyl or Het¹-carbonyl;
- alkyl: is a straight or branched saturated hydrocarbon radical having from 1 to 6 carbon atoms; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms; wherein each carbon atom can be optionally substituted with hydroxy, alkyloxy or oxo ;
- Ar: is a homocycle selected from the group of phenyl, naphthyl, acenaphthyl, tetrahydronaphthyl, each homocycle optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl;
- Het: is a monocyclic heterocycle selected from the group of *N*-phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl and benzo[1,3]dioxolyl ; each monocyclic and bicyclic heterocycle may optionally be substituted with 1, 2 or 3 substituents, each substituent independently selected from the group of halo, hydroxy, alkyl, alkyloxy, and Ar-carbonyl;
- Het¹: is a monocyclic heterocycle selected from furanyl or thienyl; or a bicyclic heterocycle selected from benzofuranyl or benzothienyl; each monocyclic and bicyclic heterocycle may optionally be substituted with 1,2 or 3 substituents, each substituent independently selected from the group of halo, alkyl and Ar;
- halo: is a substituent selected from the group of fluoro, chloro, bromo and iodo; and
- haloalkyl: is a straight or branched saturated hydrocarbon radical having from 1 to 6 carbon atoms or a cyclic saturated hydrocarbon radical having from 3 to 6 carbon atoms or a cyclic saturated hydrocarbon radical having from 3 to 6 carbon atoms attached to a straight or branched saturated hydrocarbon radical having from 1 to 6 carbon atoms; wherein one or more carbon atoms are substituted with one or more halo atoms;
optionally provided that when R¹⁰ is alkyl or benzyl, then R⁴ and R⁵ are other than hydrogen; and
optionally provided that the compound is other than

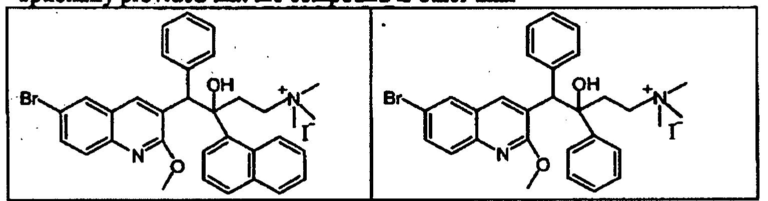

a *N*-oxide thereof, a tautomeric form thereof or a stereochemically isomeric form thereof.

A fifth interesting embodiment relates to a compound of formula (Ia) or (Ib) or any subgroup thereof as mentioned hereinbefore as interesting embodiment wherein
- A⁻: is iodide;
- R¹: is hydrogen, halo, cyano, Ar, Het, alkyl, and alkyloxy ;
- p: is an integer equal to 1, 2, 3 or 4 ; in particular 1 or 2;
- R²: is hydrogen, hydroxy, alkyloxy, alkyloxyalkyloxy, alkylthio or a radical of Formula 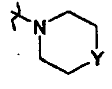 wherein Y is O ;

- R³: is alkyl, Ar, Ar-alkyl or Het;
- q: is an integer equal to zero, 1, 2, or 3 ;
- R⁴ and R⁵: each independently are hydrogen, alkyl or benzyl;
- R⁶: is hydrogen, halo or alkyl ; or
two vicinal R⁶ radicals may be taken together to form a bivalent radical of formula

-CH=CH-CH=CH-;

- r: is an integer equal to 1 ;
- R⁷: is hydrogen ;
- R⁸: is hydrogen or alkyl ;
- R⁹: is oxo ; or
- R⁸ and R⁹: together form the radical -CH=CH-N=;
- R¹⁰: is alkyl, in particular C₁₋₄alkyl;
- alkyl: is a straight or branched saturated hydrocarbon radical having from 1 to 6 carbon atoms; or is a cyclic saturated hydrocarbon radical having from 3 to 6 carbon atoms; or is a a cyclic saturated hydrocarbon radical having from 3 to 6 carbon atoms attached to a straight or branched saturated hydrocarbon radical having from 1 to 6 carbon atoms ; wherein each carbon atom can be optionally substituted with hydroxy ;
- Ar: is a homocycle selected from the group of phenyl, naphthyl, acenaphthyl and tetrahydronaphthyl, each homocycle optionally substituted with 1, 2 or 3 substituents, each substituent independently selected from the group of halo, haloalkyl, cyano, alkyloxy and morpholinyl ;
- Het: is a monocyclic heterocycle selected from the group of *N-*phenoxypiperidinyl, piperidinyl, furanyl, thienyl, pyridinyl and pyrimidinyl; or a bicyclic heterocycle selected from the group of benzothienyl, 2,3-dihydrobenzo[1,4]dioxinyl and benzo[1,3]dioxolyl; each monocyclic and bicyclic heterocycle may optionally be substituted with 1, 2 or 3 alkyl or Ar-carbonyl substituents ; and
- halo: is a substituent selected from the group of fluoro, chloro and bromo.

A sixth interesting embodiment relates to a compound of formula (Ia) or (Ib) or any subgroup thereof as mentioned hereinbefore as interesting embodiment wherein R¹ is hydrogen, halo, Ar, alkyl or alkyloxy; preferably, R¹ is halo; more preferably, R¹ is bromo.

A seventh interesting embodiment relates to a compound of formula (Ia) or (Ib) or any subgroup thereof as mentioned hereinbefore as interesting embodiment wherein p is equal to 1; preferably wherein p is equal to 1 and R¹ is other than hydrogen. Preferably, the R¹ substituent is placed in position 5, 6 or 7 of the quinoline ring, more preferably in position 6.

An eighth interesting embodiment relates to a compound of formula (Ia) or (Ib) or any subgroup thereof as mentioned hereinbefore as interesting embodiment wherein R² is hydrogen, alkyloxy or alkylthio; preferably, R² is alkyloxy, in particular C₁₋₄alkyloxy, more in particular methyloxy; or alkylthio, in particular C₁₋₄alkylthio, more in particular methylthio; more preferably, R² is alkyloxy, in particular C₁₋₄alkyloxy, more in particular methyloxy.

A ninth interesting embodiment relates to a compound of formula (Ia) or (Ib) or any subgroup thereof as mentioned hereinbefore as interesting embodiment wherein R³ is Het, Ar or Ar-alkyl, each optionally substituted with 1 or 2 substituents, that substituent preferably being a halo or haloalkyl, most preferably being a halo; preferably, R³ is Ar or Ar-alkyl, each optionally substituted with 1 or 2 substituents, that substituent preferably being a halo or haloalkyl, most preferably being a halo; more preferably, R³ is naphthyl, phenyl, naphthylC₁₋₄alkyl or phenylC₁₋₄alkyl, each optionally substituted with halo, preferably 3-fluoro; more preferably, R³ is naphthyl, phenyl or phenylC₁₋₄alkyl; preferred, R³ is naphthyl, phenyl or phenylethyl.

A tenth interesting embodiment relates to a compound of formula (Ia) or (Ib) or any subgroup thereof as mentioned hereinbefore as interesting embodiment wherein q is equal to 1, 2 or 3; preferably, q is equal to 3.

An eleventh interesting embodiment relates to a compound of formula (Ia) or (Ib) or any subgroup thereof as mentioned hereinbefore as interesting embodiment wherein R⁴ and R⁵ each independently are hydrogen or alkyl, in particular hydrogen or C₁₋₄alkyl; preferably C₁₋₄alkyl; most preferably methyl or ethyl.

A twelfth interesting embodiment relates to a compound of formula (Ia) or (Ib) or any subgroup thereof as mentioned hereinbefore as interesting embodiment wherein R⁴ and R⁵ together and including the N to which they are attached may form a radical selected from the group of pyrrolidinyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, pyridazinyl, pyrimidinyl, pyrazinyl, triazinyl, morpholinyl and thiomorpholinyl, each of said rings may optionally be substituted with alkyl, halo, haloalkyl, hydroxy, alkyloxy, amino, mono- or dialkylamino, alkylthio, alkyloxyalkyl, alkylthioalkyl, Ar-alkyl or pyrimidinyl; preferably R⁴ and R⁵ together and including the N to which they are attached may form a radical selected from the group of piperidinyl, morpholinyl or piperazinyl, each of said rings may optionally be substituted with alkyl or Ar-alkyl; or R⁴ and R⁵ together and including the N to which they are attached may form a radical selected from imidazolyl or piperidinyl.

A thirteenth interesting embodiment relates to a compound of formula (Ia) or (Ib) or any subgroup thereof as mentioned hereinbefore as interesting embodiment wherein R⁶ is hydrogen, alkyl or halo; preferably, R⁶ is hydrogen or halo; more preferably, R⁶ is hydrogen.

A fourteenth interesting embodiment relates to a compound of formula (Ia) or (Ib) or any subgroup thereof as mentioned hereinbefore as interesting embodiment wherein r is 1 or 2; preferably r is 1.

A fifteenth interesting embodiment relates to a compound of formula (Ia) or (Ib) or any subgroup thereof as mentioned hereinbefore as interesting embodiment wherein R⁷ is hydrogen or methyl; preferably R⁷ is hydrogen.

A sixteenth interesting embodiment relates to a compound of formula (Ia) or (Ib) or any subgroup thereof as mentioned hereinbefore as interesting embodiment wherein, for compounds according to Formula (Ib) only, R⁸ is alkyl, preferably methyl, and R⁹ is oxygen.

A seventeenth interesting embodiment relates to a compound of formula (Ia) or (Ib) or any subgroup thereof as mentioned hereinbefore as interesting embodiment wherein R¹⁰ is alkyl; preferably C₁₋₆alkyl; more preferably C₁₋₄alkyl.

An eighteenth interesting embodiment relates to a compound of formula (Ia) or (Ib) or any subgroup thereof as mentioned hereinbefore as interesting embodiment wherein A⁻is chloride, bromide, iodide, trifluoroacetate, acetate, triflate, sulfate, sulfonate; preferably chloide, bromide or iodide, more preferably iodide.

A nineteenth interesting embodiment relates to a compound of formula (Ia) or (Ib) or any subgroup thereof as mentioned hereinbefore as interesting embodiment wherein the compound is a compound according to formula (Ia).

A twentieth interesting embodiment relates to a compound of formula (Ia) or (Ib) or any subgroup thereof as mentioned hereinbefore as interesting embodiment wherein one or more, preferably all, of the following definitions apply:
R¹ is halo, in particular bromo;
p = 1;
R² is alkyloxy, in particular C₁₋₄alkyloxy, more in particular methoxy; or alkylthio, in particular C₁₋₄alkylthio, more in particular methylthio;
R³ is naphthyl; phenyl; phenylethyl or Het, in particular thienyl;
q = 1, 2 or 3;
R⁴ and R⁵ each independently are alkyl, in particular C₁₋₄alkyl; or R⁴ and R⁵ together and including the N to which they are attached may form a radical selected from imidazolyl or piperidinyl;
R⁶ is hydrogen or halo;
r is equal to 1;
R⁷ is hydrogen;
R¹⁰ is alkyl, in particular C₁₋₆alkyl, more in particular C₁₋₄alkyl.

A twenty first interesting embodiment is the use of a compound of formula (Ia) or (Ib) or any subgroup thereof as mentioned hereinbefore as interesting embodiment for the manufacture of a medicament for the treatment of an infection with a gram-positive and/or a gram-negative bacterium.

A twenty second interesting embodiment is the use of a compound of formula (Ia) or (Ib) or any subgroup thereof as mentioned hereinbefore as interesting embodiment for the manufacture of a medicament for the treatment of an infection with a gram-positive bacterium.

A twenty third interesting embodiment is the use of the compounds of formula (Ia) or (Ib) or any subgroup thereof as mentioned hereinbefore as interesting embodiment for the manufacture of a medicament for the treatment of an infection with a gram-negative bacterium.

A twenty fourth interesting embodiment is the use of a compound of formula (Ia) or (Ib) or any subgroup thereof as mentioned hereinbefore as interesting embodiment for the manufacture of a medicament for the treatment of a bacterial infection wherein the compound of formula (Ia) or (Ib) has a IC₉₀ < 15 µl/ml against at least one bacterium, in particular a gram-positive bacetrium, preferably a IC₉₀< 10 µl/ml, more preferably a IC₉₀ < 5 µl/ml; the IC₉₀ value being determined as described hereinafter.

Preferably, in the compounds of formula (Ia) and (Ib) or any subgroup thereof as mentioned hereinbefore as interesting embodiment, the term "alkyl" represents C₁₋₆alkyl, more preferably C₁₋₄alkyl.

Preferred compounds are selected from the following :

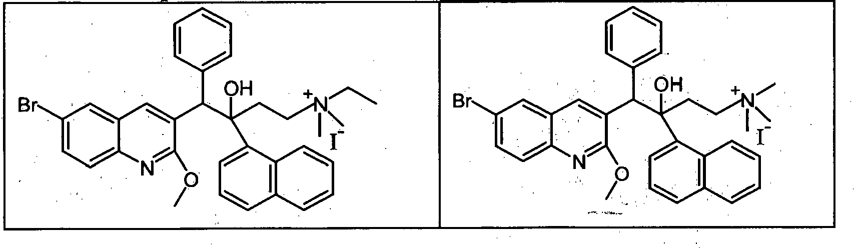

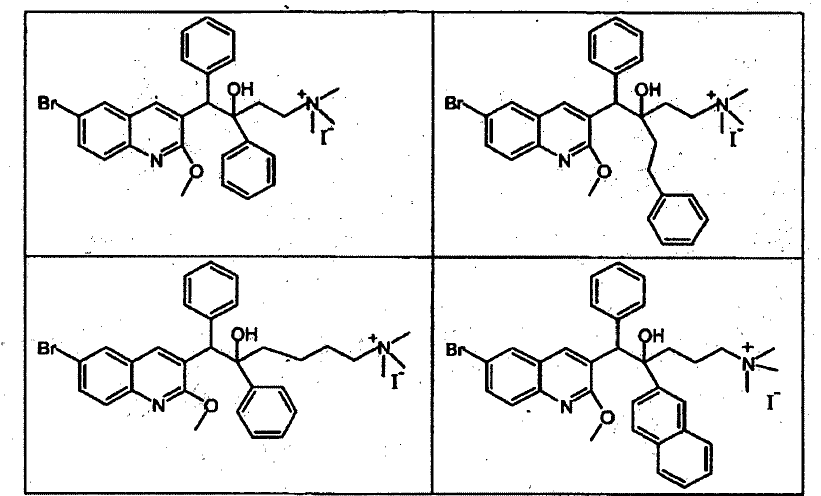

a N-oxide thereof or a stereochemically isomeric form thereof.

Especially preferred compounds are selected from compound 121, 102, 103, 10, A, E, K and R (see the Tables hereinbelow), a *N*-oxide thereof, a tautomeric form thereof or a stereochemically isomeric form thereof.

The present invention also relates to compounds A to C and E to U, a *N*-oxide thereof or a stereochemically isomeric form thereof, in particular to compounds A, E, K and R or a stereochemically isomeric form thereof.

The compounds of formula (I) can be prepared according to the methods described in WO 2004/011436. In general, the compounds according to the invention can be prepared by a succession of steps, each of which is known to the skilled person.

In particular, compounds of formula (Ia) or (Ib) can be prepared by reacting an intermediate of formula (II-a) respectively (II-b) with an intermediate of formula (III) in the presence of a suitable solvent, such as for example acetone.

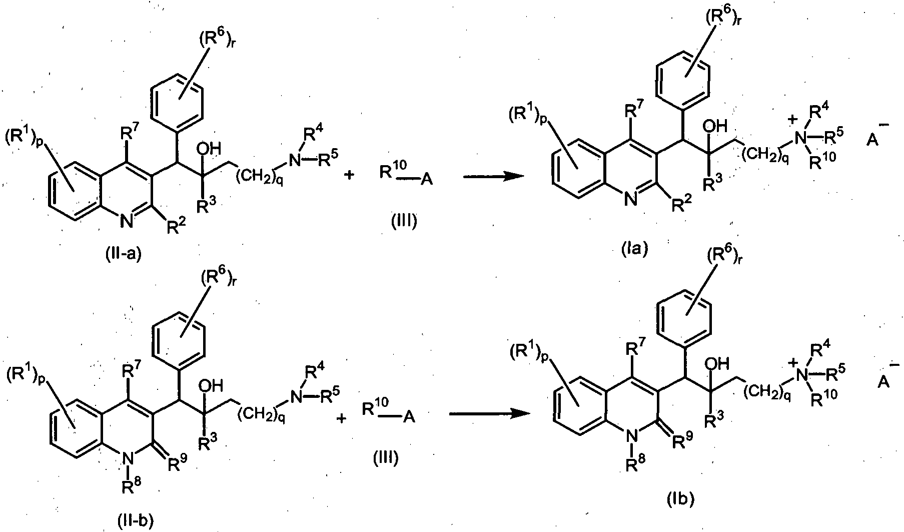

Intermediates according to formula (II-a) can be prepared by reacting an intermediate compound of formula (IV) with an intermediate compound of formula (V) according to the following reaction scheme (1) : 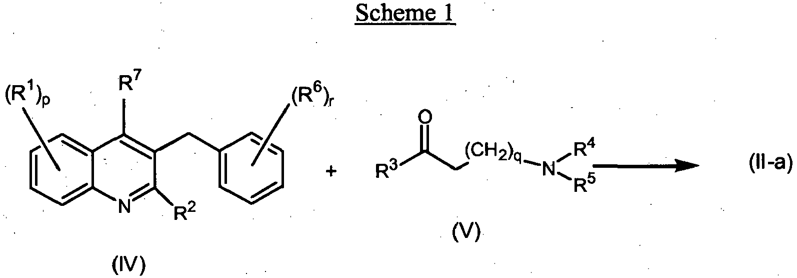 using nBuLi in a mixture of diisopropyl amine and tetrahydrofuran, wherein all variables are defined as in formula (Ia). Stirring may enhance the rate of the reaction. The reaction may conveniently be carried out at a temperature ranging between -20 and -70 °C.

The same reaction procedure can be used to synthesize intermediates of formula (II-b).

The starting materials and the intermediate compounds of formula (IV) and (V) are compounds that are either commercially available or may be prepared according to conventional reaction procedures generally known in the art. For example, intermediate compounds of formula (IV-a) or (IV-b) may be prepared according to the following reaction scheme (2): 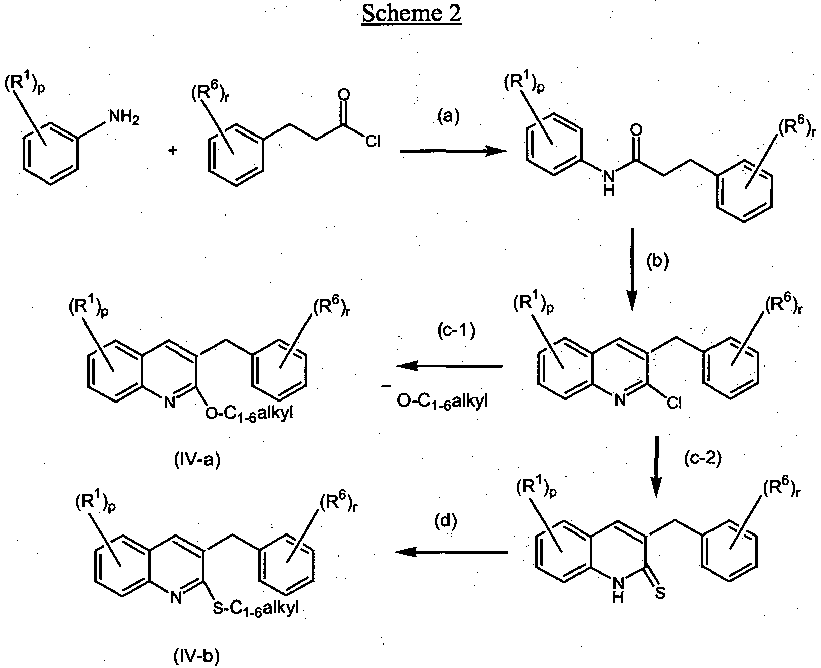 wherein all variables are defined as in formula (Ia). Reaction scheme (2) comprises step (a) in which an appropriately substituted aniline is reacted with an appropriate acylchloride such as 3-phenylpropionyl chloride, 3-fluorobenzenepropionyl chloride or *p*-chlorobenzenepropionyl chloride, in the presence of a suitable base, such as triethylamine and a suitable reaction-inert solvent, such as methylene chloride or ethylene dichloride. The reaction may conveniently be carried out at a temperature ranging between room temperature and reflux temperature. In a next step (b) the adduct obtained in step (a) is reacted with phosphoryl chloride (POCl₃) in the presence of *N,N*-dimethylformamide (Vilsmeier-Haack formylation followed by cyclization). The reaction may conveniently be carried out at a temperature ranging between room temperature and reflux temperature. In a next step (c-1), a specific R²-group, wherein R² is for example a C₁₋₆alkyloxy radical is introduced by reacting the intermediate compound obtained in step (b) with a ⁻O-C₁₋₆alkyl in the presence of a suitable solvent, such as for example HO-C₁₋₆alkyl. The intermediate compound obtained in step (b) can also be converted into an intermediate compound wherein R² is for example a C₁₋₆alkylthio radical by reaction with S=C(NH₂)₂ in the presence of a suitable solvent, such as for example an alcohol, e.g. ethanol (step (c-2)) followed by reaction with C₁₋₆alkyl-I in the presence of a suitable base, such as for example K₂CO₃ and a suitable solvent, such as for example 2-propanone.

Intermediate compounds according to formula (IV-c) may be prepared according to the following reaction scheme (3), wherein in a first step (a) an optionally substituted indole-2,3-dione is reacted with an optionally substituted 3-phenylpropionaldehyde in the presence of a suitable base such as sodium hydroxide (Pfitzinger reaction), after which the carboxylic acid compound is decarboxylated in a next step (b) at high temperature in the presence of a suitable reaction-inert solvent such as diphenylether.

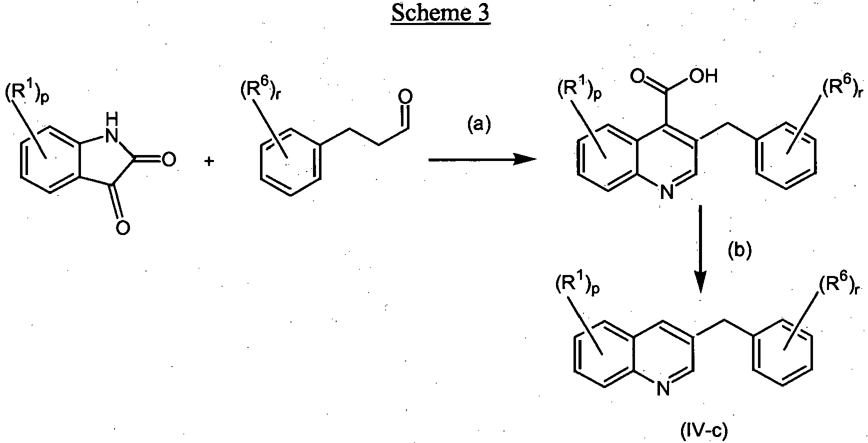

It is evident that in the foregoing and in the following reactions, the reaction products may be isolated from the reaction medium and, if necessary, further purified according to methodologies generally known in the art, such as extraction, crystallization and chromatography. It is further evident that reaction products that exist in more than one enantiomeric form, may be isolated from their mixture by known techniques, in particular preparative chromatography, such as preparative HPLC. Typically, compounds of formula (Ia) or (Ib) may be separated into their isomeric forms.

The intermediate compounds of formula (V) are compounds that are either commercially available or may be prepared according to conventional reaction procedures generally known in the art. For example, intermediate compounds of formula (V) may be prepared according to the following reaction scheme (4):

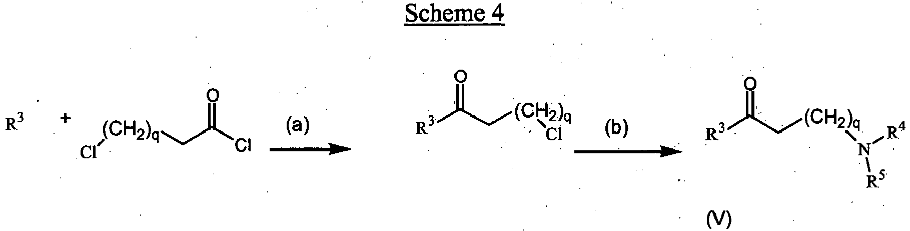

Reaction scheme (4) comprises step (a) in which R³, in particular an appropriately substituted Ar, more in particular an appropriately substituted phenyl, is reacted by Friedel-Craft reaction with an appropriate acylchloride such as 3-chloropropionyl chloride or 4-chlorobutyryl chloride, in the presence of a suitable Lewis acid, such as for example AlCl₃, FeCl₃, SnCl₄, TiCl₄ or ZnCl₂ and a suitable reaction-inert solvent, such as methylene chloride or ethylene dichloride. The reaction may conveniently be carried out at a temperature ranging between room temperature and reflux temperature. In a next step (b) an amino group (-NR⁴R⁵) is introduced by reacting the intermediate compound obtained in step (a) with a primary or secondary amine (HNR⁴R⁵).

In general, bacterial pathogens may be classified as either gram-positive or gram-negative pathogens. Antibiotic compounds with activity against both gram-positive and gram-negative pathogens are generally regarded as having a broad spectrum of activity. The compounds of the present invention are regarded as active against gram-positive and/or gram-negative bacterial pathogens. In particular, the present compounds are active against at least one gram-positive bacterium, preferably against several gram-positive bacteria, more preferably against one or more gram-positive bacteria and/or one or more gram-negative bacteria.

The present compounds have bactericidal or bacteriostatic activity.

Examples of gram-positive and gram-negative aerobic and anaerobic bacteria, include Staphylococci, for example *S. aureus;* Enterococci, for example *E. faecalis*; Streptococci, for example *S. pneumoniae, S. mutans, S. pyogens;* Bacilli, for example *Bacillus subtilis;* Listeria, for example *Listeria monocytogenes*; Haemophilus, for example *H. influenza*; Moraxella, for example *M. catarrhalis*; Pseudomonas, for example *Pseudomonas aeruginosa;* and Escherichia, for example *E. coli.* Gram-positive pathogens, for example Staphylococci, Enterococci and Streptococci are particularly important because of the development of resistant strains which are both difficult to treat and difficult to eradicate from for example a hospital environment once established. Examples of such strains are methicillin resistant *Staphylococcus aureus* (MRSA), methicillin resistant coagulase negative staphylococci (MRCNS), penicillin resistant *Streptococcus pneumoniae* and multiple resistant *Enterococcus faecium.*

The compounds of the present invention also show activity against resistant bacterial strains.

The compounds of the present invention are especially active against *Staphylococcus aureus,* including resistant *Staphylococcus aureus* such as for example methicillin resistant *Staphylococcus aureus* (MRSA), and *Streptococcus pneumoniae,* in particular against *Staphylococcus aureus.*

In particular, the compounds of the present invention are active on those bacteria of which the viability depends on proper functioning of F1F0 ATP synthase. Without being bound to any theory, it is taught that the activity of the present compounds lies in inhibition of the F1F0 ATP synthase, in particular the inhibition of the F0 complex of the F1F0 ATP synthase, more in particular the inhibition of subunit c of the F0 complex of the F1F0 ATP synthase, leading to killing of the bacteria by depletion of the cellular ATP levels of the bacteria.

Whenever used hereinbefore or hereinafter, that the compounds can treat a bacterial infection it is meant that the compounds can treat an infection with one or more bacterial strains.
Whenever used hereinbefore or hereinafter, that the bacterial infection is other than a Mycobacterial infection it is meant that the bacterial infection is other than an infection with one or more Mycobacteria strains.

The exact dosage and frequency of administration of the present compounds depends on the particular compound of formula (Ia) or (Ib) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention.

The compound of the present invention may be administered in a pharmaceutically acceptable form optionally in a pharmaceutically acceptable carrier. The compounds and compositions comprising the compounds can be administered by routes such as topically, locally or systemically. Systemic application includes any method of introducing the compound into the tissues of the body, e.g., intrathecal, epidural, intramuscular, transdermal, intravenous, intraperitoneal, subcutaneous, sublingual, rectal, and oral administration. The specific dosage of antibacterial to be administered, as well as the duration of treatment, may be adjusted as needed.

Bacterial infections which may be treated by the present compounds include, for example, central nervous system infections, external ear infections, infections of the middle ear, such as acute otitis media, infections of the cranial sinuses, eye infections, infections of the oral cavity, such as infections of the teeth, gums and mucosa, upper respiratory tract infections, lower respiratory tract infections, genitourinary infections, gastrointestinal infections, gynecological infections, septicemia, bone and joint infections, skin and skin structure infections, bacterial endocarditis, burns, antibacterial prophylaxis of surgery, and antibacterial prophylaxis in immunosuppressed patients, such as patients receiving cancer chemotherapy, or organ transplant patients.

Given the fact that the compounds of formula (Ia) or (Ib) are active against bacterial infections, the present compounds may be combined with other antibacterial agents in order to effectively combat bacterial infections.

Therefore, the present invention also relates to a combination of (a) a compound of formula (Ia) or (Ib), and (b) one or more other antibacterial agents provided that the one or more other antibacterial agents are other than antimycobacterial agents.

The present invention also relates to a combination of (a) a compound of formula (Ia) or (Ib), and (b) one or more other antibacterial agents provided that the one or more other antibacterial agents are other than antimycobacterial agents, for use as a medicine.

A pharmaceutical composition comprising a pharmaceutically acceptable carrier and, as active ingredient, a therapeutically effective amount of (a) a compound of formula (Ia) or (Ib), and (b) one or more other antibacterial agents provided that the one or more other antibacterial agents are other than antimycobacterial agents, is also comprised by the present invention.

The present invention also relates to the use of a combination or pharmaceutical composition as defined above for the treatment of a bacterial infection or for the manufacture of a medicament for the treatment of a bacterial infection.

The present pharmaceutical composition may have various pharmaceutical forms for administration purposes. As appropriate compositions there may be cited all compositions usually employed for systemically administering drugs. To prepare the pharmaceutical compositions of this invention, an effective amount of the particular compounds, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally or by parenteral injection. For example, in preparing the compositions in oral dosage form, any of the usual pharmaceutical media may be employed such as, for example, water, glycols, oils, alcohol in the case of oral liquid preparations such as suspensions, syrups, elixirs, emulsions and solutions; or solid carriers such as starches, sugars, kaolin, diluents, lubricants, binders, disintegrating agents in the case of powders, pills, capsules and tablets. Because of their ease in administration, tablets and capsules represent the most advantageous oral unit dosage forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents may be employed. Also included are solid form preparations which are intended to be converted, shortly before use, to liquid form preparations.

Depending on the mode of administration, the pharmaceutical composition will preferably comprise from 0.05 to 99 % by weight, more preferably from 0.1 to 70 % by weight of the active ingredients, and, from 1 to 99.95 % by weight, more preferably from 30 to 99.9 weight % of a pharmaceutically acceptable carrier, all percentages being based on the total composition.

The weight to weight ratio's of the compound of formula (Ia) or (Ib) and (b) the other antibacterial agent(s) when given as a combination may be determined by the person skilled in the art. Said ratio and the exact dosage and frequency of administration depends on the particular compound of formula (Ia) or (Ib) and the other antibacterial agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention.

The compounds of formula (Ia) or (Ib) and the one or more other antibacterial agents may be combined in a single preparation or they may be formulated in separate preparations so that they can be administered simultaneously, separately or sequentially. Thus, the present invention also relates to a product containing (a) a compound of formula (Ia) or (Ib), and (b) one or more other antibacterial agents provided that the one or more other antibacterial agents are other than antimycobacterial agents, as a combined preparation for simultaneous, separate or sequential use in the treatment of a bacterial infection.

The pharmaceutical composition may additionally contain various other ingredients known in the art, for example, a lubricant, stabilising agent, buffering agent, emulsifying agent, viscosity-regulating agent, surfactant, preservative, flavouring or colorant.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and segregated multiples thereof. The daily dosage of the compound according to the invention will, of course, vary with the compound employed, the mode of administration, the treatment desired and the bacterial disease indicated.

The other antibacterial agents which may be combined with the compounds of formula (I) are antibacterial agents known in the art. The other antibacterial agents comprise antibiotics of the β-lactam group such as natural penicillins, semisynthetic penicillins, natural cephalosporins, semisynthetic cephalosporins, cephamycins, 1-oxacephems, clavulanic acids, penems, carbapenems, nocardicins, monobactams; tetracyclines, anhydrotetracyclines, anthracyclines; aminoglycosides; nucleosides such as *N*-nucleosides, C-nucleosides, carbocyclic nucleosides, blasticidin S; macrolides such as 12-membered ring macrolides, 14-membered ring macrolides, 16-membered ring macrolides; ansamycins; peptides such as bleomycins, gramicidins, polymyxins, bacitracins, large ring peptide antibiotics containing lactone linkages, actinomycins, amphomycin, capreomycin, distamycin, enduracidins, mikamycin, neocarzinostatin, stendomycin, viomycin, virginiamycin; cycloheximide; cycloserine; variotin; sarkomycin A; novobiocin; griseofulvin; chloramphenicol; mitomycins; fumagillin; monensins; pyrrolnitrin; fosfomycin; fusidic acid; D-(p-hydroxyphenyl)glycine; D-phenylglycine; enediynes.

Specific antibiotics which may be combined with the present compounds of formula (Ia) or (Ib) are for example benzylpenicillin (potassium, procaine, benzathine), phenoxymethylpenicillin (potassium), phenethicillin potassium, propicillin, carbenicillin (disodium, phenyl sodium, indanyl sodium), sulbenicillin, ticarcillin disodium, methicillin sodium, oxacillin sodium, cloxacillin sodium, dicloxacillin, flucloxacillin, ampicillin, mezlocillin, piperacillin sodium, amoxicillin, ciclacillin, hectacillin, sulbactam sodium, talampicillin hydrochloride, bacampicillin hydrochloride, pivmecillinam, cephalexin, cefaclor, cephaloglycin, cefadroxil, cephradine, cefroxadine, cephapirin sodium, cephalothin sodium, cephacetrile sodium, cefsulodin sodium, cephaloridine, cefatrizine, cefoperazone sodium, cefamandole, vefotiam hydrochloride, cefazolin sodium, ceftizoxime sodium, cefotaxime sodium, cefmenoxime hydrochloride, cefuroxime, ceftriaxone sodium, ceftazidime, cefoxitin, cefmetazole, cefotetan, latamoxef, clavulanic acid, imipenem, aztreonam, tetracycline, chlortetracycline hydrochloride, demethylchlortetracycline, oxytetracycline, methacycline, doxycycline, rolitetracycline, minocycline, daunorubicin hydrochloride, doxorubicin, aclarubicin, kanamycin sulfate, bekanamycin, tobramycin, gentamycin sulfate, dibekacin, amikacin, micronomicin, ribostamycin, neomycin sulfate, paromomycin sulfate, streptomycin sulfate, dihydrostreptomycin, destomycin A, hygromycin B, apramycin, sisomicin, netilmicin sulfate, spectinomycin hydrochloride, astromicin sulfate, validamycin, kasugamycin, polyoxin, blasticidin S, erythromycin, erythromycin estolate, oleandomycin phosphate, tracetyloleandomycin, kitasamycin, josamycin, spiramycin, tylosin, ivermectin, midecamycin, bleomycin sulfate, peplomycin sulfate, gramicidin S, polymyxin B, bacitracin, colistin sulfate, colistinmethanesulfonate sodium, enramycin, mikamycin, virginiamycin, capreomycin sulfate, viomycin, enviomycin, vancomycin, actinomycin D, neocarzinostatin, bestatin, pepstatin, monensin, lasalocid, salinomycin, amphotericin B, nystatin, natamycin, trichomycin, mithramycin, lincomycin, clindamycin, clindamycin palmitate hydrochloride, flavophospholipol, cycloserine, pecilocin, griseofulvin, chloramphenicol, chloramphenicol palmitate, mitomycin C, pyrrolnitrin, fosfomycin, fusidic acid, bicozamycin, tiamulin, siccanin.

### EXPERIMENTAL PART

Of some compounds the absolute stereochemical configuration of the stereogenic carbon atom(s) therein was not experimentally determined. In those cases the stereochemically isomeric form which was first isolated is designated as "A" and the second as "B", without further reference to the actual stereochemical configuration. However, said "A" and "B" isomeric forms can be unambiguously characterized by a person skilled in the art, using art-known methods such as, for example, X-ray diffraction.

In case "A" and "B" are stereoisomeric mixtures, they can be further separated whereby the respective first fractions isolated are designated "A1" respectively "B1" and the second as "A2" respectively "B2", without further reference to the actual stereochemical configuration. However, said "A1", "A2" and "B1", "B2" isomeric forms can be unambiguously characterized by a person skilled in the art, using art-known methods such as, for example, X-ray diffraction.

For some of the compounds, stereochemical configurations are indicated in the structures. The configurations are relative configurations indicating that the groups concerned are located in the same or opposite plane of the molecule ( 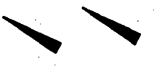 = same plane; 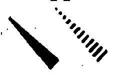 = opposite plane )

The present compounds which are also described in WO 2004/011436 bear the same compound number as in WO 2004/011436. The Ex. Nr. in the below Tables and in the synthesis protocols herein below refer to the Example numbers of WO 2004/011436 indicating according to which protocol the compounds were prepared.

Additional compounds are indicated by way of letters.

### Synthesis of compounds A, B and C

A solution of compound 15 of WO 2004/011436 (prepared according to B7) (0.1g, 0.18 mmol) and ethyl iodide (0.02 ml, 0.19 mmol) in acetone (2 ml) was stirred at 60°C for 12 hours. The solvent was evaporated and the residue was crystallized from diisopropyether and acetone. Yield: 0.02 g of compound A (diastereoisomer B) (16 %, mp=244°C).

Compound B (diastereoisomer A)was prepared according to the above protocol but starting from compound 14 of WO 2004/011436 (prepared according to B7). Yield : 71 %, mp=204°C.

Compound C (diastereoisomer B) was prepared according to the above protocol starting from compound 15 of WO 2004/011436 (prepared according to B7) and reacting this compound 15 with butyl iodide. Yield : 50 %, mp=182°C.

### Synthesis of compounds E and F

A solution of compound 95 (0.1 g, 0.187 mmol) of WO 2004/011436 (prepared according to B1) and methyl iodide (0.02 ml, 0.281 mmol) in acetone (2 ml) was stirred at room temperature for 3 hours. The solvent was evaporated and the residue was crystallized from diisopropyether. Yield : 0.115 g of compound E (diastereoisomer A) (91 %, mp>250°C).

Compound F (diastereoisomer B) was prepared according to the above protocol but starting from compound 96 of WO 2004/011436 (prepared according to B1). Yield: 87 %, mp>250°C.

### Synthesis of compound G

Compound G was prepared according to the following scheme :

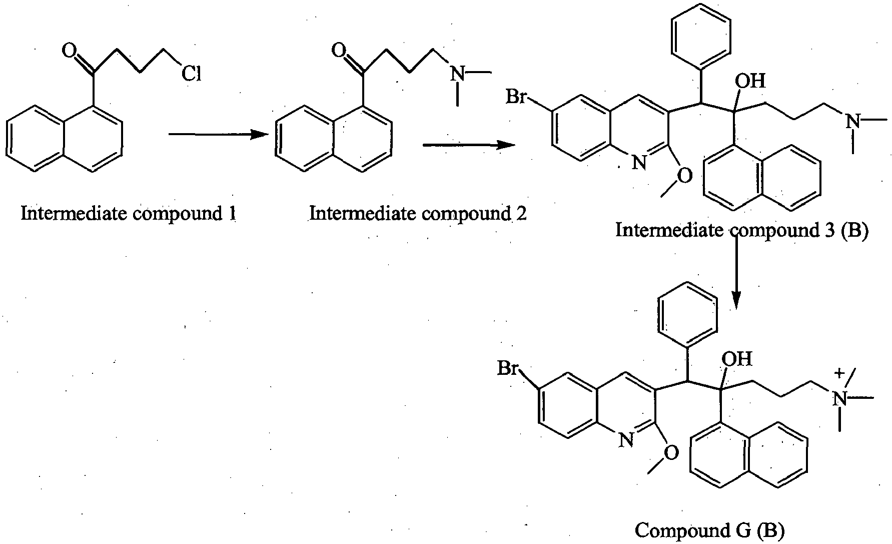

### Synthesis of intermediate compound 1

Intermediate compound 1 was prepared in the same way as intermediate compound 12 of WO 2004/011436 (according to A8) with 4-chlorobutyrylchloride. The residue (70.7 g) was purified by column chromatography over silica gel (eluent: Cyclohexane/AcOEt; 70:30; 20-45 µm). Two fractions (F1 and F2) were collected and the solvent was evaporated. F1 : 45.5 g of intermediate compound 1 (yield = 63 %).

### Synthesis of intermediate compound 2

A solution of intermediate compound 1 (2 g, 0.0086 mol), dimethylamine hydrochloride (1.4 g, 0.0172 mol) and potassium carbonate (2.4 g, 0.0174 mol) in acetonitrile (30 ml) was stirred at 80°C for 12 hours. The solvent was evaporated and the residue (2.8 g) was purified by column chromatography over silica gel (eluent: CH₂Cl₂/CH₃OH/NH₄OH; 93/7/0.5; 20-45 µm) yielding 1 g of intermediate compound 2 as an oil (yield = 49 %).

### Synthesis of intermediate compound 3

*n*BuLi 1.6M (3.3 ml, 0.0024 mol) was added slowly at -20°C under N₂ flow to a solution of diisopropylamine (0.33 ml, 0.0024 mol) in tetrahydrofuran (5 ml). The mixture was stirred at -20°C for 20 minutes, then cooled at -70°C. A solution of intermediate compound 3 of WO 2004/011436 (prepared according to A3) (0.6 g, 0.0018 mol) in tetrahydrofuran (5 ml) was added slowly. The mixture was stirred at -70°C for 1h30. A solution of present intermediate compound 2 (0.54 g, 0.0022 mol) in tetrahydrofuran (5 ml) was added slowly. The mixture was stirred at -70°C for 3 hours, hydrolyzed at -30°C with ice water, and extracted with EtOAc. The organic layer was separated, dried over MgSO₄, filtered, and the solvent was evaporated. The residue (5.3 g) was purified by column chromatography over silica gel (eluent: CH₂Cl₂/CH₃OH/NH₄OH; 99/1/0.1; 20-45 µm). Two fractions were collected and the solvent was evaporated. Fractions were crystallized separately from diisopropyether and diethylether yielding 0.04 g of diastereoisomer A (4 %) and 0.04 g of present intermediate compound 3 (diastereoisomer B) (4 %).

### Synthesis of compound G

A solution of intermediate compound 3 (0.04 g, 0.07 mmol) and methyl iodide (0.01 ml, 0.14 mmol) in acetone (2 ml) was stirred at room temperature for 2 hours. The solvent was evaporated and the residue was crystallized from diisopropyether and acetone yielding 0.047 g of compound G (diastereoisomer B) (93 %).

### Synthesis of compounds H and I

A solution of compound 197 of WO 2004/01146 (0.15g, 0.257mmol) (prepared according to B7) and methyl iodide (0.02ml, 0.257mmol) in acetone (3ml) was stirred at room temperature for 18 hours. The solvent was evaporated and the residue was crystallized from diethylether yielding 0.154g of compound I (diastereoisomer A)
(83 %, mp=188°C).

Compound H (diastereoisomer B) was prepared according to the above protocol but starting from compound 191 of WO 2004/011436 (prepared according to B7).
Yield: 0.154 g of compound H (75 %, mp=172°C).

### Synthesis of compounds K and J

A solution of compound 66 of WO 2004/011436 (0.10 g, 0.187 mmol) (prepared according to B1) and methyl iodide (0.018 ml, 0.281 mmol) in acetone (3 ml) was stirred at room temperature for 18 hours. The precipitate was filtered off, washed with acetone and dried at 70°C yielding 0.08 g of compound J (diastereoisomer B)
(53 %, mp=175°C).

Compound K (diastereoisomer A) was prepared according to the above protocol but starting from compound 65 of WO 2004/011436 (prepared according to B1). Yield : 64 % of compound K(245°C).

### Synthesis of compound L

Compound L was synthesized according to the following scheme

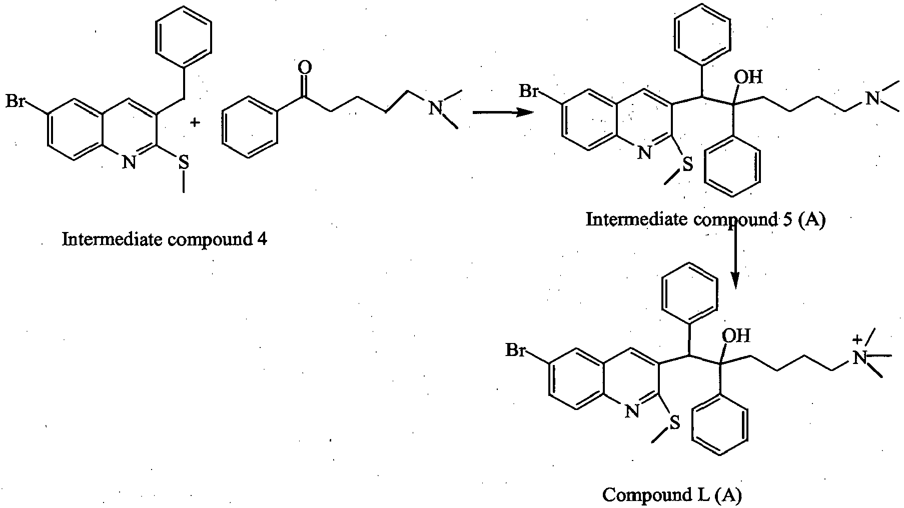

### Synthesis of intermediate compound 4

### a) Preparation of intermediate compound 4a

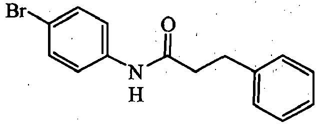

Benzenepropanoyl chloride (0.488 mol) was added dropwise at room temperature to a solution of 4-bromobenzenamine (0.407 mol) in Et₃N (70 ml) and CH₂Cl₂ (700 ml) and the mixture was stirred at room temperature overnight. The mixture was poured out into water and concentrated NH₄OH, and extracted with CH₂Cl₂. The organic layer was dried (MgSO₄), filtered, and the solvent was evaporated. The residue was crystallized from diethyl ether. The residue (119.67 g) was taken up in CH₂Cl₂ and washed with HCl 1N. The organic layer was dried (MgSO₄), filtered, and the solvent was evaporated. Yield: 107.67 g of intermediate compound 4a.

### b) Preparation of intermediate compound 4b

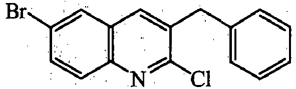

The reaction was carried out twice. POCl₃ (1.225 mol) was added dropwise at 10°C to *N,N*-dimethylformamide (0.525 mol). Then intermediate compound 4a (0.175 mol) was added at room temperature. The mixture was stirred overnight at 80°C, poured out on ice and extracted with CH₂Cl₂. The organic layer was dried (MgSO₄), filtered, and the solvent was evaporated. The product was used without further purification. Yield: 77.62 g (67 %) of intermediate compound 4b.

### c) Preparation of intermediate compound 4c

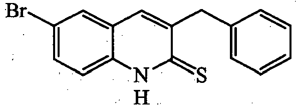

A mixture of intermediate compound 4b (0.045 mol) and thiourea (0.05 mol) in ethanol (150 ml) was stirred and refluxed for 8 hours and then brought to room temperature. A solution of KOH (0.068 mol) in water (15 ml) was added. The mixture was stirred and refluxed for 1 hour and poured out on ice. The precipitate was filtered off, washed with H₂O and dried. Yield: 11 g (74 %) of intermediate compound4c.

### d) Preparation of intermediate compound 4

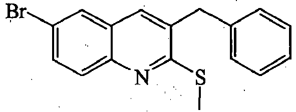

CH₃I (0.037 mol) was added slowly at room temperature to a mixture of intermediate compound 4c (0.033 mol) and K₂CO₃ (0.037 mol) in 2-propanone (150 ml). The mixture was stirred at room temperature for 8 hours, poured out into H₂O and extracted with CH₂Cl₂. The organic layer was separated, dried (MgSO₄), filtered and the solvent was evaporated. Yielding: 11.2 g. Part of this fraction (2 g) was crystallized from diethyl ether. The precipitate was filtered off and dried. Yield: 1.45 g (70 %) of intermediate compound 4.

### Synthesis of intermediate compound 5

### Preparation of intermediate compound 5

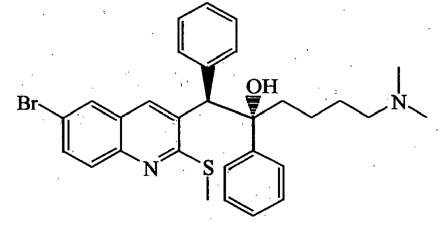

nBuLi 1.6M in hexane (0.0035 mol) was added dropwise at -20°C to a solution of *N*-(1-methylethyl)-2-propanamine (0.0035 mol) in tetrahydrofuran (7ml) under N₂ flow. The mixture was stirred at -20°C for 20 minutes, then cooled to -70°C. A solution of intermediate compound 4 (0.003 mol) in tetrahydrofuran (10 ml) was added. The mixture was stirred at -70°C for 1 hour. A solution of 5-(dimethylamino)-1-phenyl-1-pentanone (J.Am.Chem.Soc. 1972, 94(11), 3877-3883) (0.0035 mol) in tetrahydrofuran (10ml) was added. The mixture was stirred at -70°C for 3 hours. H₂O was added. The mixture was extracted with EtOAc. The organic layer was washed with saturated NaCl, dried (MgSO₄), filtered and the solvent was evaporated. The residue (2 g) was purified by column chromatography over silica gel (eluent: CH₂Cl₂/ CH₃OH/NH₄OH 94/6/0.3; 15-40 µm). Two fractions were collected and the solvent was evaporated. Residue 1 was crystallized from diisopropylether. The precipitate was filtered off and dried. Yield: (2 %) of intermediate compound 5 (diastereoisomer A).

### Synthesis of compound L

A solution of intermediate compound 5 (0.27g, 0.49 mmol) and methyl iodide (0.046 ml, 0.74 mmol) in acetone (5 ml) was stirred at room temperature for 24 hours. The solvent was evaporated, the residue was taken up in diisopropylether/ CH₂Cl₂, the precipitate was filtered off, washed with diisopropylether and dried at 70°C yielding 0.17 g of compound L (diastereoisomer A) (51 %, mp=233°C).

### Synthesis of compound U

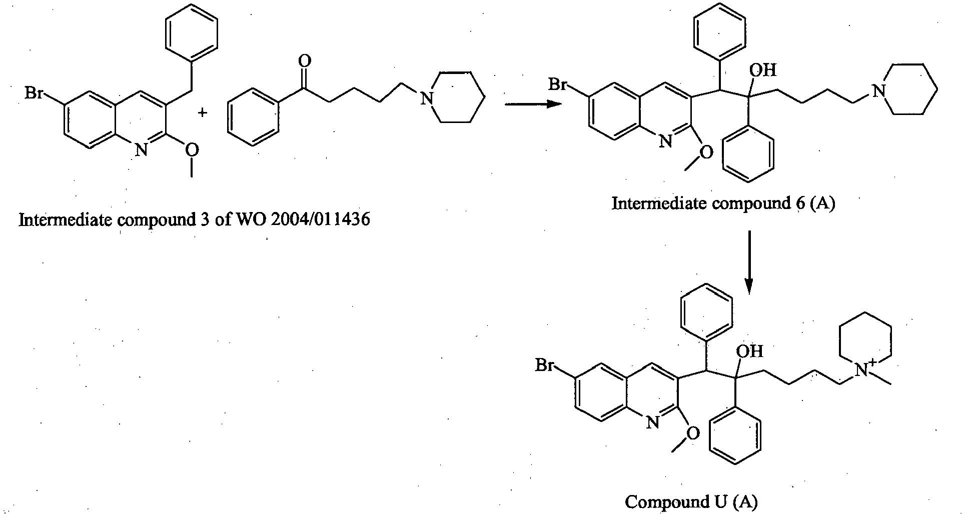

### Synthesis of intermediate compound 6

nBuLi 1.6M (1.15 ml, 1.83 mmol) was added slowly at -20°C under N₂ flow to a solution of diisopropylamine (0.256 ml, 1.83 mmol) in tetrahydrofuran (4 ml). The mixture was stirred at -20°C for 20 minutes, then cooled at -70°C. A solution of intermediate compound 3 of WO 2004/011436 (0.5 g, 1.52 mmol) in tetrahydrofuran (5 ml) was added slowly. The mixture was stirred at -70°C for 1 hour. A solution of 1-phenyl-5-(1-piperidinyl)-1-pentanone (0.45 g, 1.83 mmol) in tetrahydrofuran (5 ml) was added slowly. The mixture was stirred at -70°C for 1.5 hours, hydrolyzed at -70°C with water, and extracted with EtOAc. The organic layer was separated, washed with brine, dried over MgSO₄, filtered, and the solvent was evaporated. The residue (0.9 g) was purified by column chromatography over silica gel (eluent: CH₂Cl₂/CH₃OH/NH₄OH; 97/3/0.1; kromasil 10µm). Two fractions were collected and the solvent was evaporated. The first product fraction yielded intermediate compound 6 (A). Yield: 0.085 g of intermediate compound 6 (diastereoisomer A) (10 %, mp=129°C). The second product fraction was crystallized from diisopropylether to give diastereoisomer B (yield = 6 %, mp=166°C).

### Synthesis of compound U

A solution of intermediate compound 6 (A) (0.020 g, 0.035 mmol) and methyl iodide (0.0032 ml, 0.052 mmol) in acetone (2 ml) was stirred at room temperature for 24 hours. The precipitate was filtered off, washed with diethylether and dried at 60°C. Yield: 0.014g of compound U (diastereoisomer A) (55%, mp=170°C).

### Synthesis of compounds Rand S

A solution of compound 126 of WO 2004/011436 (0.2 g, 0.3 mmol) and methyl iodide (0.0558 g, 0.3 mmol) in acetone (5 ml) was stirred at room temperature for 24 hours. The mixture was evaporated till dryness and then crystallized from diisopropylether and acetone. Yielding : 0.147 g of compound R (B) (95 %, mp=224°C).

Compound S (diastereoisomer A) was prepared according to the above protocol but starting from compound 125 of WO 2004/011436. Yield : 0.069 g of compound S (65 %, mp=214°C).

### Synthesis of compounds M, N, O, P, Q and T

Compound M (A) was prepared according to the protocol of compound R but starting from compound 24 of WO 2004/011436. Yield: 0.025 g of compound M (A) (43 %).

Compound N (B) was prepared according to the protocol of compound R but starting from compound 37 of WO 2004/011436. Yield: 0.048 g of compound N (B) (85 %). Compound O (B) was prepared according to the protocol of compound R but starting from compound 39 of WO 2004/011436. Yield: 0.043 g of compound O (B) (75 %).

Compound P (B) was prepared according to the protocol of compound but starting from compound 50 of WO 2004/011436. Yield: 0.032 g of compound P (B) (56 %).

Compound Q (A) was prepared according to the protocol of compound R but starting from compound 45 of WO 2004/011436. Yield: 0.149 g of compound Q (A) (91 %).

Compound T (A) was prepared according to the protocol of compound R but starting from compound 32 of WO 2004/011436. Yield: 0.038 g of compound T (A) (66 %).

Tables 1 and 2 list compounds of formula (Ia) or (Ib) according to the present invention.

**Table 1:**

| | | | | | |
|---|---|---|---|---|---|
| 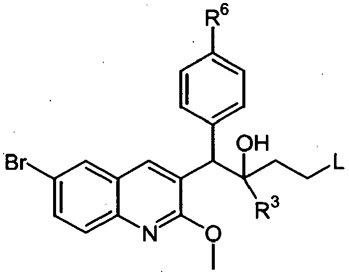 | | | | | |

| Comp nr. | Ex. nr. | R⁶ | R³ | L | Stereochemis try and melting points |
|---|---|---|---|---|---|
| A | | H | 1-naphthyl | 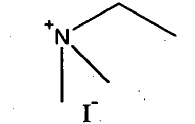 | (B); 244°C |
| C | | H | 1-naphthyl | 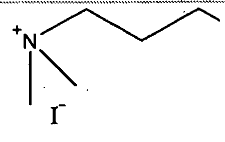 | (B); 182°C |
| 121 | B5 | H | 1-naphthyl | 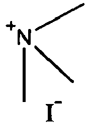 | (A1); 210°C |
| B | | H | 1-naphthyl | 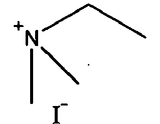 | (A); 204°C |
| 103 | B5 | H | 1-naphthyl | 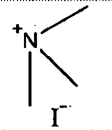 | (B); >250°C |
| E | | H | phenylCH₂-CH₂- | 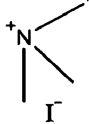 | (A); >250°C |
| F | | H | phenylCH₂-CH₂- | 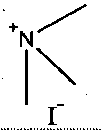 | (B); >250°C |
| 102 | B5 | H | 1-naphthyl | 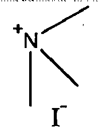 | (A2); 210°C |
| 57 | B5 | H | phenyl | 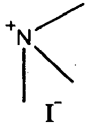 | (A); 244°C |
| 10 | B5 | H | phenyl | 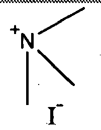 | (B); 198°C |
| M | | H | 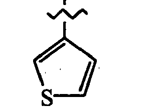 | 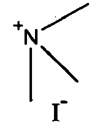 | (A); 268°C |
| N | | Cl | phenyl | 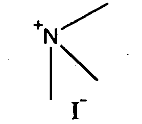 | (B); 255°C |
| O | | H | 3-fluorophenyl | 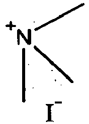 | (B); 184°C |
| P | | H | phenyl | 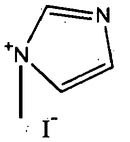 | (B); 246°C |
| Q | | H | 2-naphthyl | 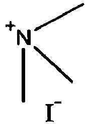 | (A) |

**Table 2:**

| | | | | | |
|---|---|---|---|---|---|
| 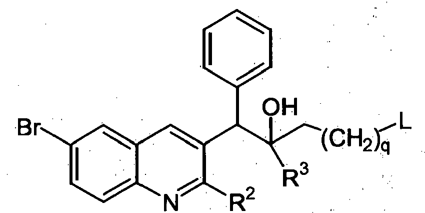 | | | | | |

| Comp. nr. | R² | R³ | q | L | Stereochemistry and melting points |
|---|---|---|---|---|---|
| G | OCH₃ | 1-naphthyl | 2 | 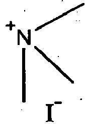 | (B) |
| H | OCH₃ | 2-naphthyl | 3 | 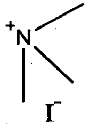 | (B); 172°C |
| K | OCH₃ | phenyl | 3 | 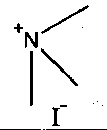 | (A); 245°C |
| J | OCH₃ | phenyl | 3 | 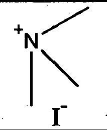 | (B); 175°C |
| I | OCH₃ | 2-naphthyl | 3 | 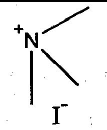 | (A); 188°C |
| L | SCH₃ | phenyl | 3 | 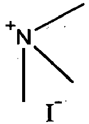 | (A); 233°C |
| R | OCH₃ | 2-naphthyl | 2 | 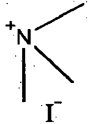 | (B); 224°C |
| S | OCH₃ | 2-naphthyl | 2 | 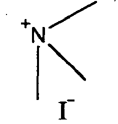 | (A); 214°C |
| T | SCH₃ | phenyl | 1 | 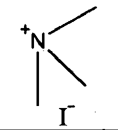 | (A); 266°C |
| U | OCH₃ | phenyl | 3 | 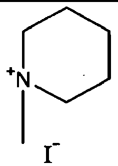 | (A); 170°C |

### Analytical methods

### General method

The HPLC gradient was supplied by an Alliance HT 2795 (Waters) system consisting of a quaternary pump with degasser, an autosampler, and DAD detector. Flow from the column was split to the MS detector. MS detectors were configured with an electrospray ionization source. The capillary needle voltage was 3 kV and the source temperature was maintained at 100 °C. Nitrogen was used as the nebulizer gas. Data acquisition was performed with a Waters-Micromass MassLynx-Openlynx data system.

### LCMS-method 1

In addition to the general procedure: LCMS analysis was carried out (electrospray ionization in both positive and negative (pulsed) mode scanning from 100 to 1000 amu) on a Sunfire C18 column (Waters, Milford, MA; 3.5 µm, 4.6 x 100 mm) with a flow rate of 0.8 ml/minute. Two mobile phases (mobile phase A: 35% 6.5 mM ammonium acetate + 30 % acetonitrile +35 % formic acid (2 ml/l); mobile phase B: 100 % acetonitrile) were employed to run a gradient condition from 100 % A for 1 minute to 100 % B in 4 minutes, 100 % B at a flow rate of 1.2 ml/minute for 4 minutes to 100 % A at 0.8 ml/minute in 3 minutes, and reequilibrate with 100 % A for 1.5 minute.

The mass of compound G (without counter ion) was recorded with LCMS-method 1 (liquid chromatography mass spectrometry). The parent peak (MH+) is 583.

### LCMS-method 2

In addition to the general procedure: Reversed phase HPLC was carried out on an Kromasil C18 column (5 µm, 4.6 x 150 mm) with a flow rate of 1.0 ml/min. Three mobile phases (mobile phase A: 100 % 7 mM ammonium acetate; mobile phase B: 100 % acetonitrile; mobile phase C: 0.2 % formic acid + 99.8 % ultra-pure Water) were employed to run a gradient condition from 30 % A, 40% B and 30% C (hold for 1 minute) to 100 % B in 4 minutes, 100% B for 5 minutes and reequilibrate with initial conditions for 3 minutes. An injection volume of 5 µl was used.

Cone voltage was 20 V for positive ionization mode. Mass spectra were acquired by scanning from 100 to 900 in 0.8 seconds using an interscan delay of 0.08 seconds.

The mass of compound Q (without counter ion) was recorded with LCMS-method 2 (liquid chromatography mass spectrometry). The parent peak (MH+) is 569. The retention time (Rₜ) is 6.20.

### Pharmacological examples

### Preparation of bacterial suspensions for susceptibility testing:

The bacteria used in this study were grown overnight in flasks containing 100 ml Mueller-Hinton Broth (Becton Dickinson - cat. no. 275730) in sterile de-ionized water, with shaking, at 37 °C. Stocks (0.5 ml/tube) were stored at -70 °C until use. Bacteria titrations were performed in microtiter plates and colony forming units (CFUs) were determined. In general, an inoculum level of approximately 100 CFUs was used for susceptibility testing.

### Anti bacterial Susceptibility testing: IC₉₀ determination

### Microtitre plate assay

Flat-bottom, sterile 96-well plastic microtiter plates were filled with 180 µl of sterile deionized water, supplemented with 0.25 % BSA. Subsequently, stock solutions (7.8 x final test concentration) of compounds were added in 45 µl volumes in column 2. Serial five-fold dilutions (45 µl in 180 µl) were made directly in the microtiter plates from column 2 to reach column 11. Untreated control samples with (column 1) and without (column 12) inoculum were included in each microtiter plate. Depending on the bacteria type, approximately 10 to 60 CFU per well of bacteria inoculum (100 TCID50), in a volume of 100 µl in 2.8x Mueller-Hinton broth medium, was added to the rows A to H, except column 12. The same volume of broth medium without inoculum was added to column 12 in row A to H. The cultures were incubated at 37°C for 24 hours under a normal atmosphere (incubator with open air valve and continuous ventilation). At the end of incubation, one day after inoculation, the bacterial growth was quantitated fluorometrically. Therefore resazurin (0.6 mg/ml) was added in a volume of 20 µl to all wells 3 hours after inoculation, and the plates were re-incubated overnight. A change in colour from blue to pink indicated the growth of bacteria.

The fluorescence was read in a computer-controlled fluorometer (Cytofluor Biosearch) at an excitation wavelength of 530 nm and an emission wavelength of 590 nm. The % growth inhibition achieved by the compounds was calculated according to standard methods. The IC₉₀ (expressed in µg/ml) was defined as the 90 % inhibitory concentration for bacterial growth. The results are shown in Table 3.

### Agar dilution method.

MIC₉₉ values (the minimal concentration for obtaining 99 % inhibition of bacterial growth) can be determined by performing the standard Agar dilution method according to NCCLS standards* wherein the media used includes Mueller-Hinton agar.
* Clinical laboratory standard institute. 2005. Methods for dilution Antimicrobial susceptibility tests for bacteria that grows Aerobically: approved standard -sixth edition

### Time kill assays

Bactericidal or bacteriostatic activity of the compounds may be determined in a time kill assay using the broth microdilution method *. In a time kill assay on *Staphylococcus aureus* and methicillin resistant *S. aureus* (MRSA), the starting inoculum *of S. aurues* and MRSA is 10⁶ CFU / ml in Muller Hinton broth. The antibacterial compounds are used at the concentration of 0.1 to 10 times the MIC (i.e. IC₉₀ as determined in microtitre plate assay). Wells receiving no antibacterial agent constitute the culture growth control. The plates containing the microorganism and the test compounds are incubated at 37 °C. After 0, 4, 24, and 48 hrs of incubation samples are removed for determination of viable counts by serial dilution (10⁻¹ to 10⁻⁶) in sterile PBS and plating (200 µl) on Mueller Hinton agar. The plates are incubated at 37 °C for 24 hrs and the number of colonies are determined. Killing curves can be constructed by plotting the log₁₀CFU per ml versus time. A bactericidal effect is commonly defined as 3-log₁₀ decrease in number of CFU per ml as compared to untreated inoculum. The potential carryover effect of the drugs is removed by serial dilutions and counting the colonies at highest dilution used for plating. No carryover effect is observed at the dilution of 10⁻² used for plating. This results in limit of detection 5 X 10² CFU / ml or <2.7 log CFU/ml.
***** Zurenko,G.E. et al. In vitro activities of U-100592 and U-100766, novel oxazolidinone antibacterial agents. Antimicrob. Agents Chemother. 40, 839-845 (1996).

### Results

A time kill assay was performed with compound 102 and the control drug ciprofloxacin.

Compound 102 demonstrated bactericidal activity on S. aureus, as did the control antibiotic ciprofloxacin. Bactericidal activities were observed at 1 and 10 times MIC90 (1 and 10 X MIC equals to 2.3 and 23 ug/ml for compound 102). At 0.1 times the MIC, the treated samples followed the control in growth.

Also for MRSA, compound 12 demonstrated marked bactericidal activity as compared to ciprofloxacin for which these strains have developed resistance. MRSA is resistant not only to methicillin but also to flouroquinolines like ciprofloxacin and as such no bactericidal effect was observed using this drug. On MRSA at 24 hours compound 12 was mostly bacteriostatic but after 48 hours it showed marked reduction in viable counts.

### Determination of cellular ATP levels

In order to analyse the change in the total cellular ATP concentration (using ATP bioluminescence Kit, Roche), assays are carried out by growing a culture *of S. aureus* (ATCC29213) stock in 100 ml Mueller Hinton flasks and incubate in a shaker-incubator for 24 hrs at 37 °C (300 rpm). Measure OD₄₀₅ nm and calculate the CFU/ml. Dilute the cultures to 1 x 10⁶ CFU/ml (final concentration for ATP measurement: 1 x 10⁵ CFU/100 µl per well) and add test compound at 0.1 to 10 times the MIC (i.e. IC₉₀ as determined in microtitre plate assay). Incubate these tubes for 0, 30 and 60 minutes at 300 rpm and 37°C. Use 0.6 ml bacterial suspension from the snap-cap tubes and add to a new 2 ml eppendorf tubes. Add 0.6 ml cell lysis reagent (Roche kit), vortex at max speed and incubate for 5 minutes at room temperature. Cool on ice. Let the luminometer warm up to 30°C (Luminoskan Ascent Labsystems with injector). Fill one column (= 6 wells) with 100 µl of the same sample. Add 100 µl Luciferase reagent to each well by using the injector system. Measure the luminescence for 1 sec.

**Table 3 : IC₉₀ values (µg/ml) determined according to the Microtitre plate assay.**

| | IC90 (µg/ml) | | | | | | | | | | | | | |
|---|---|---|---|---|---|---|---|---|---|---|---|---|---|---|
| Comp. No. | BSU | ECO | ECO | EFA | EFA | LMO | PAE | SMU | SPN | SPY | STA | STA | STA | STA |
| | 43639 | 25922 | 35218 | 14506 | 29212 | 49594 | 27853 | 33402 | 6305 | 8668 | 43300 | 25923 | 29213 | RMETH |
| A | | | | | | | | | | | | | 0.4 | |
| B | | | | | | | | | | | | | 0.4 | |
| 121 | 2.8 | 12.4 | | 2.8 | 2.8 | 0.5 | 13.9 | 2.8 | 3.5 | 3.5 | 3.5 | 2.2 | 0.4 | 2.2 |
| C | | | | | | | | | | | | | 0.4 | |
| G | | | | | | | | | | | | | 0.4 | |
| H | | | | | | | | | | | | | 0.5 | |
| 103 | 2.8 | 11.1 | 17.5 | 2.8 | 2.8 | 0.5 | 13.9 | 2.8 | 3.5 | 2.8 | 2.8 | 2.2 | 2.0 | 2.2 |
| E | | | | | | | | | | | | | 2.1 | |
| F | | | | | | | | | | | | | 2.1 | |
| K | | | | | | | | | | | | | 2.1 | |
| J | | | | | | | | | | | | | 2.1 | |
| 102 | 2.8 | 13.9 | | 2.8 | 2.8 | 2.2 | 13.9 | 2.5 | 3.1 | 2.5 | 3.5 | 2.2 | 2.8 | 2.8 |
| I | | | | | | | | | | | | | 2.9 | |
| 57 | 2.6 | | | 12.9 | 12.9 | 2.6 | | 12.9 | 16.3 | 12.9 | 12.9 | 12.9 | 12.9 | 12.9 |
| 10 | 10.3 | | | 12.9 | 5.8 | 1.2 | | 10.3 | 16.3 | 1.3 | 5.8 | 11.5 | 14.5 | 8.2 |
| L | 2.2 | | 11 | | 2.2 | 2.2 | 2.2 | 0.4 | 2.2 | 0.4 | | | 2.2 | |
| M | | | | | | | | | 8.34 | 8.34 | | | 1.7 | |
| N | | | 17.55 | | 1.75 | | 9.87 | | 1.75 | 1.75 | | | 1.8 | |
| O | | | | | 8.53 | | | | 1.7 | 1.7 | | | 1.9 | |
| P | | | | | 10.84 | | 10.84 | | 2.16 | 2.16 | | | 2.2 | |
| Q | | | | | 1.8 | | 4.53 | | 1.8 | 1.8 | | | 1.8 | |
| R | | | 9.27 | | 1.17 | | 2.07 | | 0.46 | 0.74 | | | 0.4 | |
| S | | | | | 1.85 | | 1.85 | | 0.47 | 1.85 | | | 0.4 | |
| U | | | | | 1.86 | | 9.33 | | 2.09 | 1.86 | | | 1.9 | |
| T | | | | | 3.8 | | 8.5 | | 1.7 | 1.7 | | | 1.7 | |

BSU 43639 means *Bacillus subtilis* (ATCC43639); ECO 25922 means *Escherichia coli* (ATCC25922); ECO 35218 means *Escherichia coli* (ATCC35218);EFA 14506 means *Enterococcus faecalis* (ATCC14506); EFA 29212 means *Enterococcus faecalis* (ATCC29212); LMO 49594 means *Listeria monocytogenes* (ATCC49594); PAE 27853 means *Pseudomonas aeruginosa* (ATCC27853); SMU 33402 means *Streptococcus mutans* (ATCC33402); SPN 6305 means *Streptococcus pneumoniae* (ATCC6305); SPY 8668 means *Streptococcus pyogens* (ATCC8668); STA 43300 means *Staphylococcus aureus* (ATCC43300); STA 25923 means *Staphylococcus aureus* (ATCC25923); STA 29213 means *Staphylococcus aureus* (ATCC29213); STA RMETH means methicilline resistant *Staphylococcus aureus* (MRSA) (a clinical isolate from the University of Antwerp).
ATCC means American type tissue culture.

## Claims

1. Use of a compound for the manufacture of a medicament for the treatment of a bacterial infection caused by *Staphylococci, Enterococci* or *Streptococci,* said compound being a compound of formula (Ia) or (Ib) 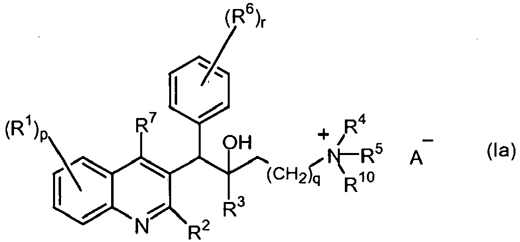 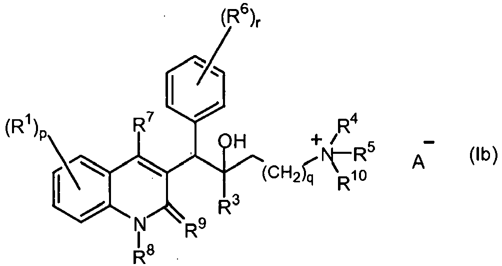 a *N-*oxide thereof, a tautomeric form thereof or a stereochemically isomeric form thereof wherein
A- is a pharmaceutically acceptable counter ion;
R¹ is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
p is an integer equal to 1, 2, 3 or 4 ;
R² is hydrogen, hydroxy, mercapto, alkyloxy, alkyloxyalkyloxy, alkylthio, mono or di(alkyl)amino or a radical of formula 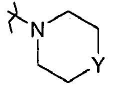 wherein Y is
R³ CH₂, O, S, NH or *N*-alkyl; is alkyl, Ar, Ar-alkyl, Het or Het-alkyl;
q is an integer equal to zero, 1, 2, 3 or 4 ;
R⁴ and R⁵ each independently are hydrogen, alkyl or benzyl;
R⁴ and R⁵ together and including the N to which they are attached may form a radical selected from the group of pyrrolidinyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, pyridazinyl, pyrimidinyl, pyrazinyl, triazinyl, morpholinyl and thiomorpholinyl, each of said rings may optionally be substituted with alkyl, halo, haloalkyl, hydroxy, alkyloxy, amino, mono- or dialkylamino, alkylthio, alkyloxyalkyl, alkylthioalkyl, Ar-alkyl or pyrimidinyl;
R⁶ is hydrogen, halo, haloalkyl, hydroxy, Ar, alkyl, alkyloxy, alkylthio, alkyloxyalkyl, alkylthioalkyl, Ar-alkyl or di(Ar)alkyl ; or
two vicinal R⁶ radicals may be taken together to form a bivalent radical of formula
-CH=CH-CH=CH-;
r is an integer equal to 1, 2, 3, 4 or 5 ;
R⁷ is hydrogen, alkyl, Ar or Het ;
R⁸ is hydrogen or alkyl ;
R⁹ is oxo ; or
R⁸ and R⁹ together form the radical -CH=CH-N=;
R¹⁰ is alkyl, alkylcarbonyl, Ar, Ar-alkyl, Ar-carbonyl, Het¹-alkyl or Het¹-carbonyl;
alkyl is a straight or branched saturated hydrocarbon radical having from 1 to 6 carbon atoms ; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with hydroxy, alkyloxy or oxo ;
Ar is a homocycle selected from the group of phenyl, naphthyl, acenaphthyl, tetrahydronaphthyl, each homocycle optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl;
Het is a monocyclic heterocycle selected from the group of *N*-phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl and benzo[1,3]dioxolyl ; each monocyclic and bicyclic heterocycle may optionally be substituted with 1, 2 or 3 substituents, Het¹ each substituent independently selected from the group of halo, hydroxy, alkyl, alkyloxy, and Ar-carbonyl; is a monocyclic heterocycle selected from furanyl or thienyl; or a bicyclic heterocycle selected from benzofuranyl or benzothienyl; each monocyclic and bicyclic heterocycle may optionally be substituted with 1, 2 or 3 substituents, each substituent independently selected from the group of halo, alkyl and Ar;
halo is a substituent selected from the group of fluoro, chloro, bromo and iodo; and
haloalkyl is a straight or branched saturated hydrocarbon radical having from 1 to 6 carbon atoms or a cyclic saturated hydrocarbon radical having from 3 to 6 carbon atoms or a cyclic saturated hydrocarbon radical having from 3 to 6 carbon atoms attached to a straight or branched saturated hydrocarbon radical having from 1 to 6 carbon atoms; wherein one or more carbon atoms are substituted with one or more halo atoms.

2. Use according to claim 1 wherein the compound of formula (Ia) or (Ib) is a compound having the following formula 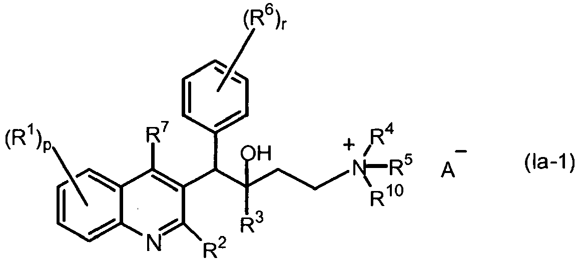 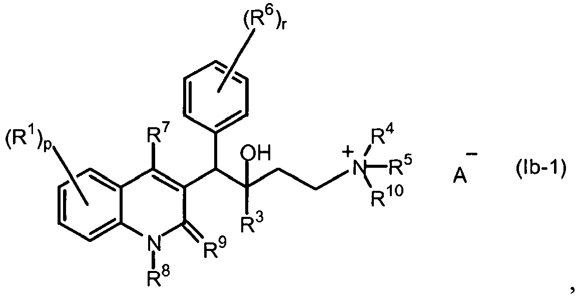 a *N*-oxide thereof, a tautomeric form thereof or a stereochemically isomeric form thereof.

3. Use according to claim 1 wherein the compound of formula (Ia) or (lb) is a compound having the following formula 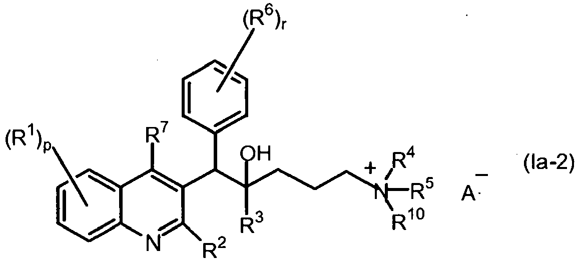 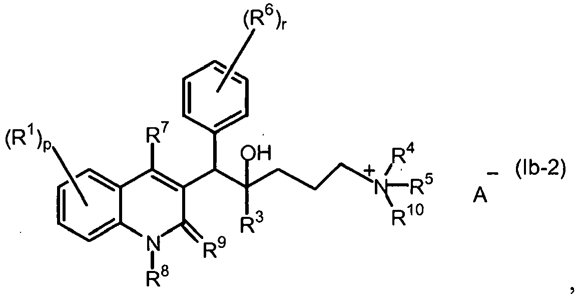 a *N*-oxide thereof, a tautomeric form thereof or a stereochemically isomeric form thereof.

4. Use according to claim 1 wherein the compound of formula (Ia) or (Ib) is a compound having the following formula 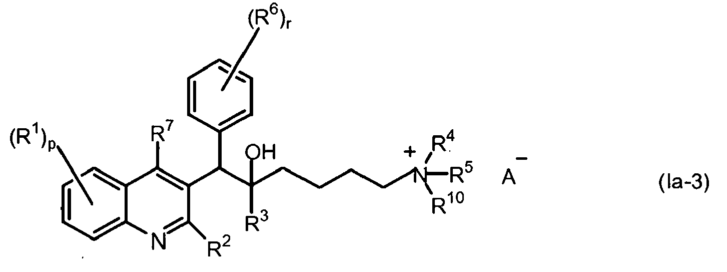 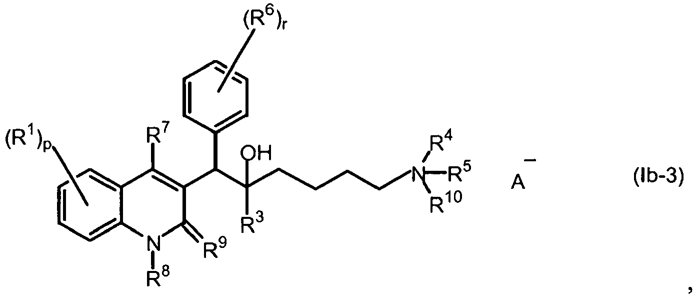 a *N*-oxide thereof, a tautomeric form thereof or a stereochemically isomeric form thereof.

5. Use according to any one of the preceding claims wherein R¹ is halo.

6. Use according to any one of the preceding claims wherein p is equal to 1.

7. Use according to any one of the preceding claims wherein R² is alkyloxy or alkylthio.

8. Use according to claim 7 wherein R² is C₁₋₄alkyloxy.

9. Use according to any one of the preceding claims wherein R³ is Het, Ar or Ar-alkyl.

10. Use according to claim 9 wherein R³ is Ar or Ar-alkyl.

11. Use according to claim 9 wherein R³ is thienyl, naphthyl, phenyl, naphthylC₁₋₄alkyl or phenylC₁₋₄alkyl.

12. Use according to claim 11 wherein R³ is naphthyl, phenyl or phenylC₁₋₄alkyl.

13. Use according to any one of the preceding claims wherein R⁴ and R⁵ are C₁₋₄alkyl or R⁴ and R⁵ together and including the N to which they are attached may form a radical selected from imidazolyl or piperidinyl.

14. Use according to claim 13 wherein R⁴ and R⁵ are C₁₋₄alkyl.

15. Use according to any one of the preceding claims wherein R⁶ is hydrogen or halo.

16. Use according to any one of the preceding claims wherein r is equal to 1.

17. Use according to any one of the preceding claims wherein R⁷ is hydrogen.

18. Use according to any one of the preceding claims wherein R¹⁰ is alkyl.

19. Use according to claim 18 wherein R¹⁰ is C₁₋₆alkyl.

20. Use according to any one of the preceding claims wherein A- is iodide.

21. Use according to any one of the preceding claims wherein the compound is a compound according to formula (Ia).

22. Use of a compound of formula (Ia) according to claim 1 wherein R¹ is halo; p = 1; R² is alkyloxy or alkylthio; R³ is naphthyl, phenyl, phenylethyl or thienyl; q = 1, 2 or 3; R⁴ and R⁵ are C₁₋₄alkyl or R⁴ and R⁵ together and including the N to which they are attached may form a radical selected from imidazolyl or piperidinyl; R⁶ is hydrogen or halo; r is equal to 1; R⁷ is hydrogen; R¹⁰ is C₁₋₆alkyl; A⁻ is iodide.

23. Use according to claim 1 wherein the compound is selected from the following compounds 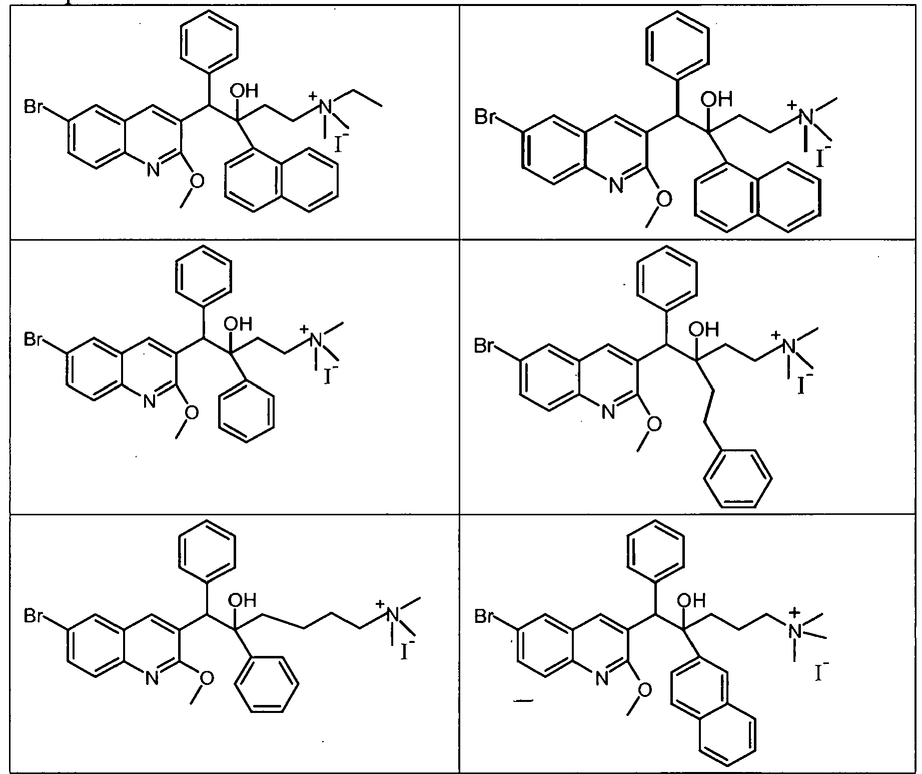 a *N*-oxide thereof or a stereochemically isomeric form thereof.

24. Use according to any one of the preceding claims wherein the bacterial infection is an infection with a gram-positive bacterium.

25. A compound of formula (Ia) or (Ib) 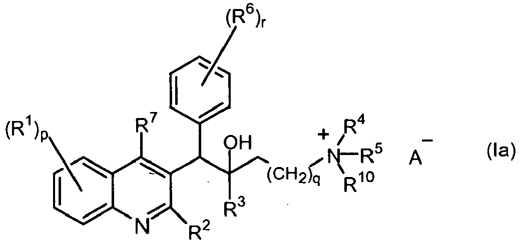 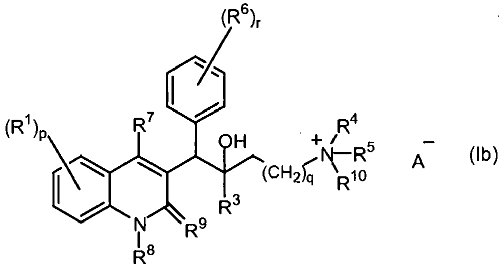 a *N*-oxide thereof, a tautomeric form thereof or a stereochemically isomeric form thereof wherein R¹, p, R², R³, q, R⁴, R⁵, R⁶, r, R⁷, R⁸, R⁹, R¹⁰ and A- are defined as in claim 1;
provided that when R¹⁰ is alkyl or benzyl, then R⁴ and R⁵ are other than hydrogen; and provided that the compound is other than 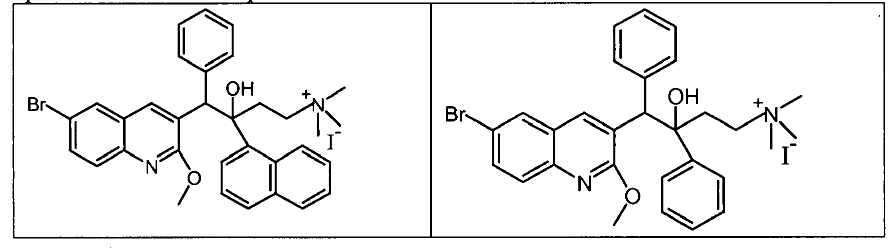 a *N*-oxide thereof, a tautomeric form thereof or a stereochemically isomeric form thereof.

26. A compound according to claim 25 wherein the compound is selected from: 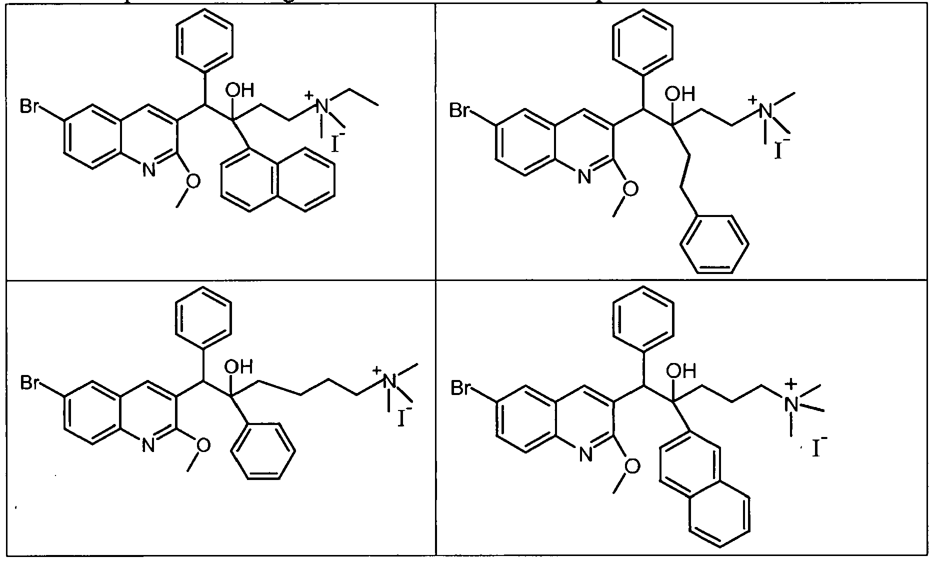 a *N*-oxide thereof or a stereochemically isomeric form thereof.

27. A compound according to claim 26 wherein the compound is selected from :
| | |
|---|---|
| 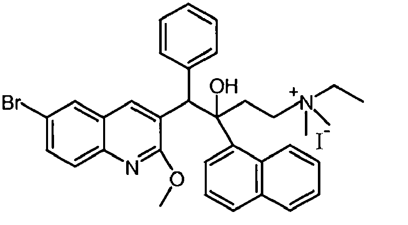 | 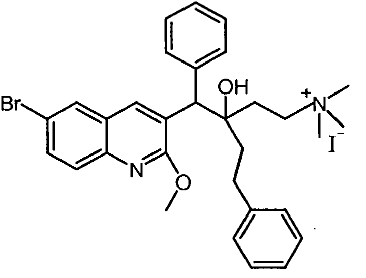 |
| (diastereoisomer B) | (diastereoisomer A) |
| 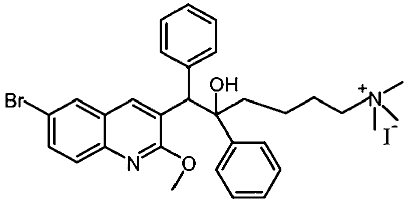 | 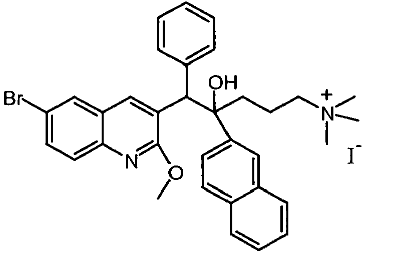 |
| (diastereoisomer A) | (diastereoisomer B) |
or a stereochemically isomeric form thereof, wherein the stereochemically isomeric form which was first isolated is designated as "A" and the second as "B".

28. A combination of (a) a compound of formula (Ia) or (Ib) as defined in any one of the preceding claims, and (b) one or more other antibacterial agents provided that the one or more other antibacterial agents are other than antimycobacterial agents, wherein the combination is for use in the treatment of a bacterial infection caused by *Staphylococci, Enterococci* or *Streptococci.*

29. A pharmaceutical composition comprising a pharmaceutically acceptable carrier and, as active ingredient, a therapeutically effective amount of (a) a compound of formula (Ia) or (lb) as defined in any one of claims 1 to 27, and (b) one or more other antibacterial agents provided that the one or more other antibacterial agents are other than antimycobacterial agents, wherein the composition is for use in the treatment of a bacterial infection caused by *Staphylococci, Enterococci* or *Streptococci.*

30. A compound as defined in any one of claims 1 to 27, for use in the treatment of a bacterial infection caused by *Staphylococci, Enterococci* or *Streptococci.*

31. A product containing (a) a compound of formula (Ia) or (lb) as defined in any one of claims 1 to 27, and (b) one or more other antibacterial agents provided that the one or more other antibacterial agents are other than antimycobacterial agents, as a combined preparation for simultaneous, separate or sequential use in the treatment of a bacterial infection caused by *Staphylococci, Enterococci* or *Streptococci.*

32. Use as claimed in any of claims 1 to 24, combination as claimed in claim 28, composition as claimed in claim 29, compound as claimed in claim 30 or product as claimed in claim 31, wherein the bacterial infection is an infection with methicillin resistant *Staphylococcus aureus* (MRSA), methicillin resistant coagulase negative staphylococci (MRCNS), penicillin resistant *Streptococcus pneumoniae* or multiple resistant *Enterococcus faecium.*

33. Use, combination, composition, compound or product as claimed in claim 32, wherein the bacterial infection is an infection with *Staphylococcus aureus* or *Streptococcus pneumoniae*.

34. Use, combination, composition, compound or product as claimed in claim 33, wherein the bacterial infection is an infection with *Staphylococcus aureus* (MRSA).

## Patentansprüche

1. Verwendung einer Verbindung zur Herstellung eines Medikaments zur Behandlung einer durch *Staphylococci, Enterococci* oder *Streptococci* verursachten bakteriellen Infektion, wobei es sich bei der Verbindung um eine Verbindung der Formel (Ia) oder (Ib) 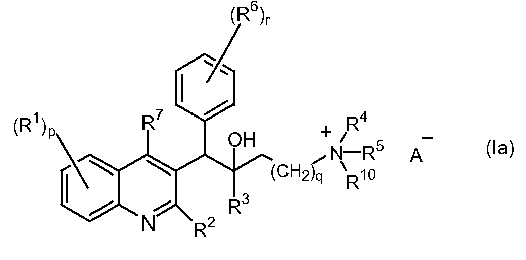 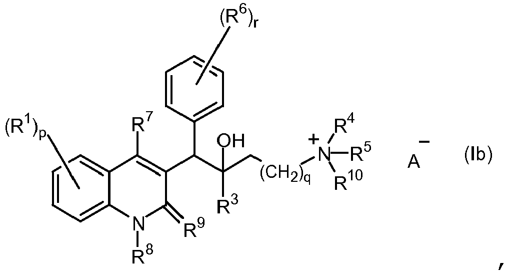 ein N-Oxid davon, eine tautomere Form davon oder eine stereochemisch isomere Form davon handelt, wobei
A⁻ für ein pharmazeutisch unbedenkliches Gegenion steht,
R¹ für Wasserstoff, Halogen, Halogenalkyl, Cyano, Hydroxy, Ar, Het, Alkyl, Alkyloxy, Alkylthio, Alkyloxyalkyl, Alkylthioalkyl, Ar-Alkyl oder Di(Ar)alkyl steht,
p für eine ganze Zahl gleich 1, 2, 3 oder 4 steht,
R² für Wasserstoff, Hydroxy, Mercapto, Alkyloxy, Alkyloxyalkyloxy, Alkylthio, Mono- oder Di(alkyl)amino oder einen Rest der Formel 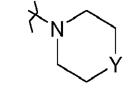
R³ steht, wobei Y für CH₂, O, S, NH oder N-Alkyl steht, für Alkyl, Ar, Ar-Alkyl, Het oder Het-Alkyl steht,
q für eine ganze Zahl gleich Null, 1, 2, 3 oder 4 steht,
R⁴ und R⁵ unabhängig voneinander für Wasserstoff, Alkyl oder Benzyl stehen,
R⁴ und R⁵ zusammen und einschließlich des N, an den sie gebunden sind, einen aus der aus Pyrrolidinyl, 2-Pyrrolinyl, 3-Pyrrolinyl, Pyrrolyl, Imidazolidinyl, Pyrazolidinyl, 2-Imidazolinyl, 2-Pyrazolinyl, Imidazolyl, Pyrazolyl, Triazolyl, Piperidinyl, Pyridinyl, Piperazinyl, Pyridazinyl, Pyrimidinyl, Pyrazinyl, Triazinyl, Morpholinyl und Thiomorpholinyl bestehenden Gruppe ausgewählten Rest stehen, wobei diese Ringe jeweils gegebenenfalls durch Alkyl, Halogen, Halogenalkyl, Hydroxy, Alkyloxy, Amino, Mono- oder Dialkylamino, Alkylthio, Alkyloxyalkyl, Alkylthioalkyl, Ar-Alkyl oder Pyrimidinyl substituiert sein können,
R⁶ für Wasserstoff, Halogen, Halogenalkyl, Hydroxy, Ar, Alkyl, Alkyloxy, Alkylthio, Alkyloxyalkyl, Alkylthioalkyl, Ar-Alkyl oder Di(Ar)alkyl steht oder
zwei vicinale R⁶-Reste zusammen einen zweiwertigen Rest
der Formel -CH=CH-CH=CH- bilden können,
r für eine ganze Zahl gleich 1, 2, 3, 4 oder 5 steht,
R⁷ für Wasserstoff, Alkyl, Ar oder Het steht,
R⁸ für Wasserstoff oder Alkyl steht,
R⁹ für Oxo steht oder
R⁸ und R⁹ zusammen den Rest -CH=CH-N= bilden,
R¹⁰ für Alkyl, Alkylcarbonyl, Ar, Ar-Alkyl, Ar-Carbonyl, Het¹-Alkyl oder Het¹-Carbonyl steht, Alkyl für einen geradkettigen oder verzweigten gesättigten Kohlenwasserstoffrest mit 1 bis 6 Kohlenstoffatomen steht oder für einen cyclischen gesättigten Kohlenwasserstoffrest mit 3 bis 6 Kohlenstoffatomen steht oder für einen cyclischen gesättigten Kohlenwasserstoffrest mit 3 bis 6 Kohlenstoffatomen gebunden an einen geradkettigen oder verzweigten gesättigten Kohlenwasserstoffrest mit 1 bis 6 Kohlenstoffatomen steht, wobei die Kohlenstoffatome jeweils gegebenenfalls durch Hydroxy, Alkyloxy oder Oxo substituiert sein können,
Ar für einen aus der aus Phenyl, Naphthyl, Acenaphthyl und Tetrahydronaphthyl bestehenden Gruppe ausgewählten Homocyclus steht, wobei die Homocyclen jeweils gegebenenfalls durch 1, 2 oder 3 Substituenten substituiert sein können, wobei die Substituenten jeweils unabhängig voneinander aus der aus Hydroxy, Halogen, Cyano, Nitro, Amino, Mono- oder Dialkylamino, Alkyl, Halogenalkyl, Alkyloxy, Halogenalkyloxy, Carboxyl, Alkyloxycarbonyl, Aminocarbonyl, Morpholinyl und Mono- oder Dialkylaminocarbonyl bestehenden Gruppe ausgewählt sind,
Het für einen aus der aus N-Phenoxypiperidinyl, Piperidinyl, Pyrrolyl, Pyrazolyl, Imidazolyl, Furanyl, Thienyl, Oxazolyl, Isoxazolyl, Thiazolyl, Isothiazolyl, Pyridinyl, Pyrimidinyl, Pyrazinyl und Pyridazinyl bestehenden Gruppe ausgewählten monocyclischen Heterocyclus oder einen aus der aus Chinolinyl, Chinoxalinyl, Indolyl, Benzimidazolyl, Benzoxazolyl, Benzisoxazolyl, Benzothiazolyl, Benzisothiazolyl, Benzofuranyl, Benzothienyl, 2,3-Dihydrobenzo[1,4]dioxinyl und Benzo[1,3]dioxolyl bestehenden Gruppe ausgewählten bicyclischen Heterocyclus steht, wobei die monocyclischen und bicyclischen Heterocyclen jeweils gegebenenfalls durch 1, 2 oder 3 Substituenten substituiert sein können, wobei die Substituenten jeweils unabhängig voneinander aus der aus Halogen, Hydroxy, Alkyl, Alkyloxy und Ar-Carbonyl bestehenden Gruppe ausgewählt sind,
Het¹ für einen aus Furanyl und Thienyl ausgewählten monocyclischen Heterocyclus oder einen aus Benzofuranyl und Benzothienyl ausgewählten bicyclischen Heterocyclus steht, wobei die monocyclischen und bicyclischen Heterocyclen jeweils gegebenenfalls durch 1, 2 oder 3 Substituenten substituiert sein können, wobei die Substituenten jeweils unabhängig voneinander aus der aus Halogen, Alkyl und Ar bestehenden Gruppe ausgewählt sind,
Halogen für einen aus der aus Fluor, Chlor, Brom und Iod bestehenden Gruppe ausgewählten Substituenten steht und
Halogenalkyl für einen geradkettigen oder verzweigten gesättigten Kohlenwasserstoffrest mit 1 bis 6 Kohlenstoffatomen oder einen cyclischen gesättigten Kohlenwasserstoffrest mit 3 bis 6 Kohlenstoffatomen oder einen cyclischen gesättigten Kohlenwasserstoffrest mit 3 bis 6 Kohlenstoffatomen gebunden an einen geradkettigen oder verzweigten gesättigten Kohlenwasserstoffrest mit 1 bis 6 Kohlenstoffatomen steht, wobei ein oder mehrere Kohlenstoffatome durch ein oder mehrere Halogenatome substituiert sind.

2. Verwendung nach Anspruch 1, wobei es sich bei der Verbindung der Formel (Ia) bzw. (Ib) um eine Verbindung mit der folgenden Formel 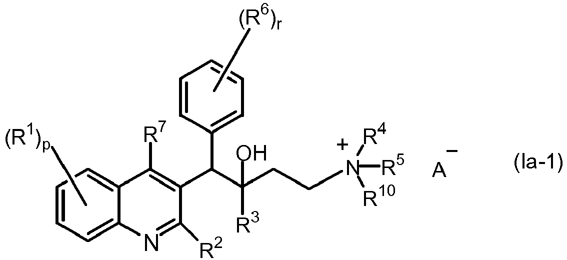 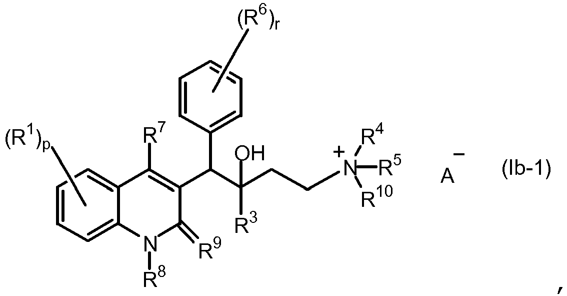 ein N-Oxid davon, eine tautomere Form davon oder eine stereochemisch isomere Form davon handelt.

3. Verwendung nach Anspruch 1, wobei es sich bei der Verbindung der Formel (Ia) bzw. (Ib) um eine Verbindung mit der folgenden Formel 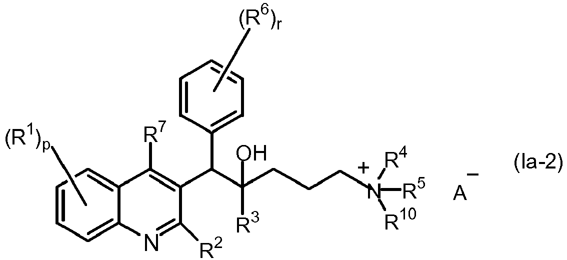 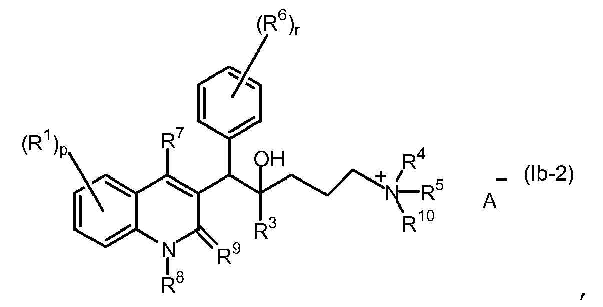 ein N-Oxid davon, eine tautomere Form davon oder eine stereochemisch isomere Form davon handelt.

4. Verwendung nach Anspruch 1, wobei es sich bei der Verbindung der Formel (Ia) bzw. (Ib) um eine Verbindung mit der folgenden Formel 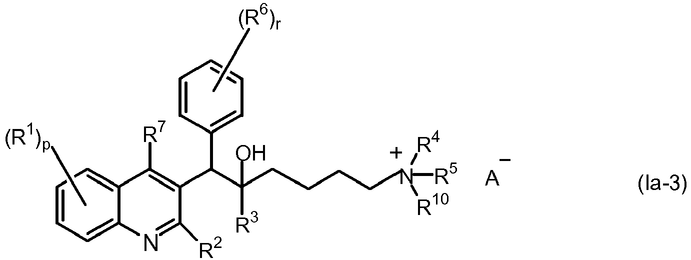 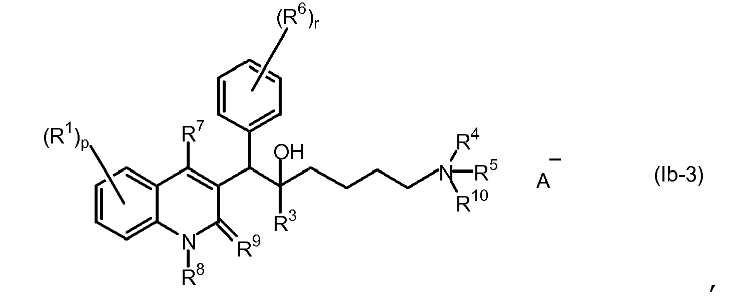 ein N-Oxid davon, eine tautomere Form davon oder eine stereochemisch isomere Form davon handelt.

5. Verwendung nach einem der vorhergehenden Ansprüche, wobei R¹ für Halogen steht.

6. Verwendung nach einem der vorhergehenden Ansprüche, wobei p gleich 1 ist.

7. Verwendung nach einem der vorhergehenden Ansprüche, wobei R² für Alkyloxy oder Alkylthio steht.

8. Verwendung nach Anspruch 7, wobei R² für C₁₋₄-Alkyloxy steht.

9. Verwendung nach einem der vorhergehenden Ansprüche, wobei R³ für Het, Ar oder Ar-Alkyl steht.

10. Verwendung nach Anspruch 9, wobei R³ für Ar oder Ar-Alkyl steht.

11. Verwendung nach Anspruch 9, wobei R³ für Thienyl, Naphthyl, Phenyl, Naphthyl-C₁₋₄-alkyl oder Phenyl-C₁₋₄-alkyl steht.

12. Verwendung nach Anspruch 11, wobei R³ für Naphthyl, Phenyl oder Phenyl-C₁₋₄-alkyl steht.

13. Verwendung nach einem der vorhergehenden Ansprüche, wobei R⁴ und R⁵ für C₁₋₄-Alkyl stehen oder R⁴ und R⁵ zusammen und einschließlich des N, an den sie gebunden sind, einen aus Imidazolyl und Piperidinyl ausgewählten Rest bilden können.

14. Verwendung nach Anspruch 13, wobei R⁴ und R⁵ für C₁₋₄-Alkyl stehen.

15. Verwendung nach einem der vorhergehenden Ansprüche, wobei R⁶ für Wasserstoff oder Halogen steht.

16. Verwendung nach einem der vorhergehenden Ansprüche, wobei r gleich 1 ist.

17. Verwendung nach einem der vorhergehenden Ansprüche, wobei R⁷ für Wasserstoff steht.

18. Verwendung nach einem der vorhergehenden Ansprüche, wobei R¹⁰ für Alkyl steht.

19. Verwendung nach Anspruch 18, wobei R¹⁰ für C₁₋₆-Alkyl steht.

20. Verwendung nach einem der vorhergehenden Ansprüche, wobei A⁻ für Iodid steht.

21. Verwendung nach einem der vorhergehenden Ansprüche, wobei es sich bei der Verbindung um eine Verbindung gemäß Formel (Ia) handelt.

22. Verwendung einer Verbindung der Formel (Ia) nach Anspruch 1, wobei R¹ für Halogen steht, p = 1, R² für Alkyloxy oder Alkylthio steht, R³ für Naphthyl, Phenyl, Phenylethyl oder Thienyl steht, q = 1, 2 oder 3, R⁴ und R⁵ für C₁₋₄-Alkyl stehen oder R⁴ und R⁵ zusammen und einschließlich des N, an den sie gebunden sind, einen aus Imidazolyl und Piperidinyl ausgewählten Rest bilden können, R⁶ für Wasserstoff oder Halogen steht, r gleich 1 ist, R⁷ für Wasserstoff steht, R¹⁰ für C₁₋₆-Alkyl steht, A⁻für Iodid steht.

23. Verwendung nach Anspruch 1, wobei die Verbindung ausgewählt ist aus den folgenden Verbindungen 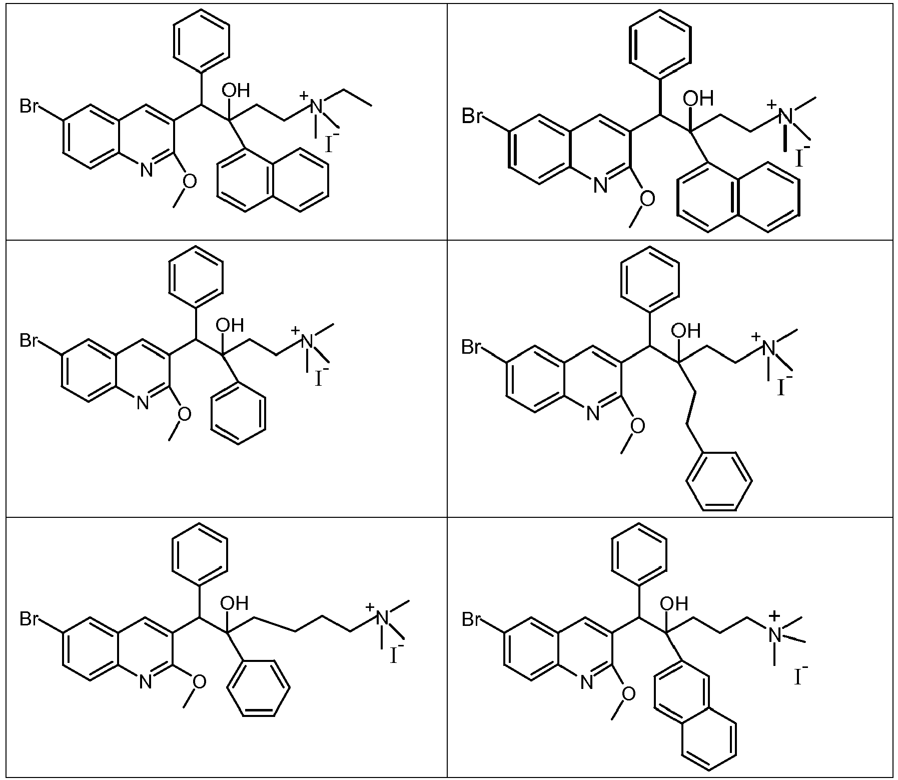 einem N-Oxid davon oder einer stereochemisch isomeren Form davon.

24. Verwendung nach einem der vorhergehenden Ansprüche, wobei es sich bei der bakteriellen Infektion um eine Infektion mit einem grampositiven Bakterium handelt.

25. Verbindung der Formel (Ia) oder (Ib) 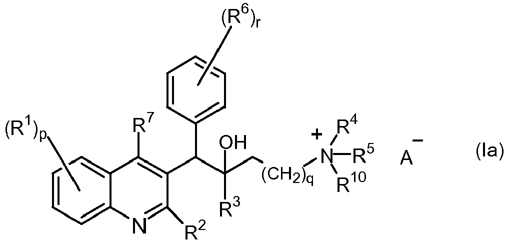 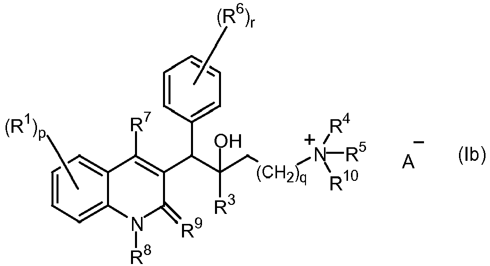 ein N-Oxid davon, eine tautomere Form davon oder eine stereochemisch isomere Form davon, wobei R¹, p, R², R³, q, R⁴, R⁵, R⁶, r, R⁷, R⁸, R⁹, R¹⁰ und A⁻wie in Anspruch 1 definiert sind,
mit der Maßgabe, dass, wenn R¹⁰ für Alkyl oder Benzyl steht, R⁴ und R⁵ nicht für Wasserstoff stehen, und
mit der Maßgabe, dass es sich bei der Verbindung nicht um 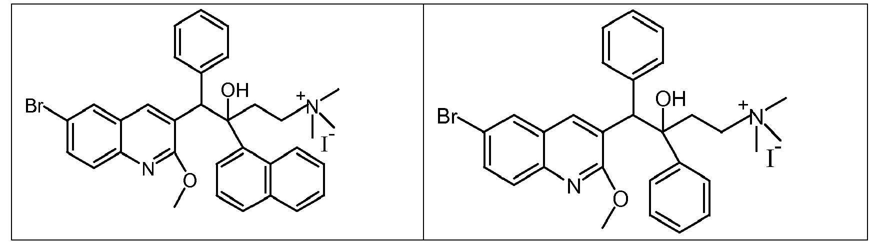 ein N-Oxid davon, eine tautomere Form davon oder eine stereochemisch isomere Form davon handelt.

26. Verbindung nach Anspruch 25, wobei die Verbindung ausgewählt ist aus: 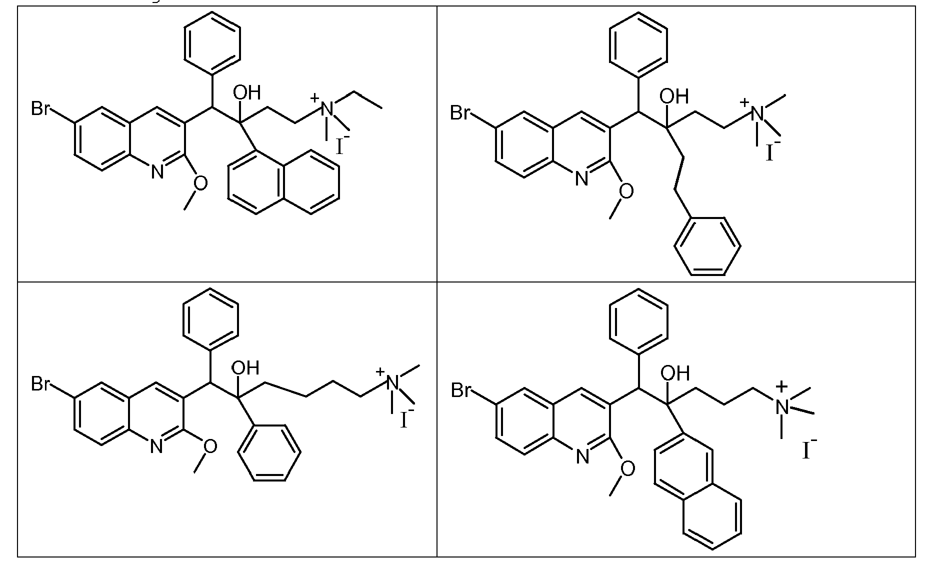 einem N-Oxid davon oder einer stereochemisch isomeren Form davon.

27. Verbindung nach Anspruch 26, wobei die Verbindung ausgewählt ist aus:
| | |
|---|---|
| 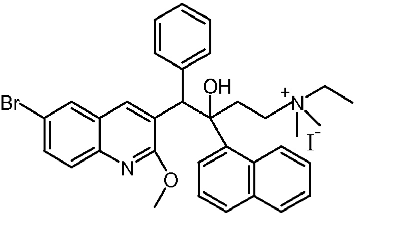 | 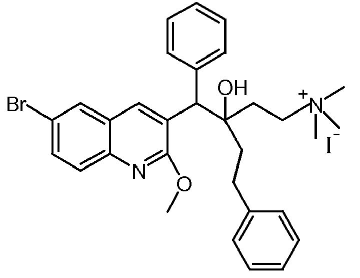 |
| (Diastereoisomer B) | (Diastereoisomer A) |
| 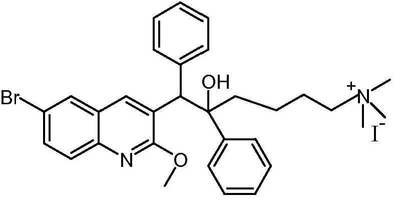 | 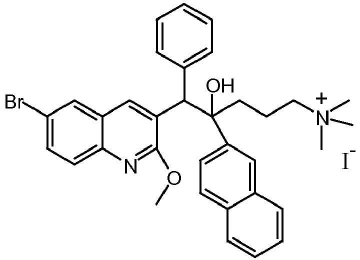 |
| (Diastereoisomer A) | (Diastereoisomer B) |
oder einer stereochemisch isomeren Form davon, wobei die zuerst isolierte stereochemisch isomere Form als "A" und die zweite als "B" bezeichnet wird.

28. Kombination von (a) einer wie in einem der vorhergehenden Ansprüche definierten Verbindung der Formel (Ia) oder (Ib) und (b) eines oder mehrerer anderer antibakterieller Mittel, mit der Maßgabe, dass es sich bei dem einen oder den mehreren anderen antibakteriellen Mitteln nicht um antimykobakterielle Mittel handelt, wobei die Kombination für die Verwendung bei der Behandlung einer durch *Staphylococci, Enterococci* oder *Streptococci* verursachten bakteriellen Infektion bestimmt ist.

29. Pharmazeutische Zusammensetzung, umfassend einen pharmazeutisch unbedenklichen Träger und, als Wirkstoff, eine therapeutisch wirksame Menge von (a) einer wie in einem der Ansprüche 1 bis 27 definierten Verbindung der Formel (Ia) oder (Ib) und (b) eines oder mehrerer anderer antibakterieller Mittel, mit der Maßgabe, dass es sich bei dem einen oder den mehreren antibakteriellen Mitteln nicht um antimykobakterielle Mittel handelt, wobei die Zusammensetzung für die Verwendung bei der Behandlung einer durch *Staphylococci, Enterococci* oder *Streptococci* verursachten bakteriellen Infektion bestimmt ist.

30. Verbindung, definiert wie in einem der Ansprüche 1 bis 27, zur Verwendung bei der Behandlung einer durch *Staphylococci, Enterococci* oder *Streptococci* verursachten bakteriellen Infektion.

31. Produkt, enthaltend (a) eine wie in einem der Ansprüche 1 bis 27 definierte Verbindung der Formel (Ia) oder (Ib) und (b) ein oder mehrere andere antibakterielle Mittel, mit der Maßgabe, dass es sich bei dem einen oder den mehreren anderen antibakteriellen Mitteln nicht um antimykobakterielle Mittel handelt, als Kombinationspräparat zur gleichzeitigen, getrennten oder aufeinanderfolgenden Anwendung bei der Behandlung einer durch *Staphylococci, Enterococci* oder *Streptococci* verursachten bakteriellen Infektion.

32. Verwendung nach einem der Ansprüche 1 bis 24, Kombination nach Anspruch 28, Zusammensetzung nach Anspruch 29, Verbindung nach Anspruch 30 oder Produkt nach Anspruch 31, wobei es sich bei der bakteriellen Infektion um eine Infektion mit Methicillin resistentem *Staphylococcus aureus* (MRSA), Methicillin resistenten koagulasenegativen Staphylococci (MRCNS), Penicillin resistentem *Streptococcus pneumoniae* oder *Enterococcus faecium* mit multipler Resistenz handelt.

33. Verwendung, Kombination, Zusammensetzung, Verbindung oder Produkt nach Anspruch 32, wobei es sich bei der bakteriellen Infektion um eine Infektion mit *Staphylococcus aureus* oder *Streptococcus pneumoniae* handelt.

34. Verwendung, Kombination, Zusammensetzung, Verbindung oder Produkt nach Anspruch 33, wobei es sich bei der bakteriellen Infektion um eine Infektion mit *Staphylococcus aureus* (MRSA) handelt.

## Revendications

1. Utilisation d'un composé pour la fabrication d'un médicament pour le traitement d'une infection bactérienne causée par des staphylocoques, des entérocoques ou des streptocoques, ledit composé étant un composé de formule (la) ou (Ib) 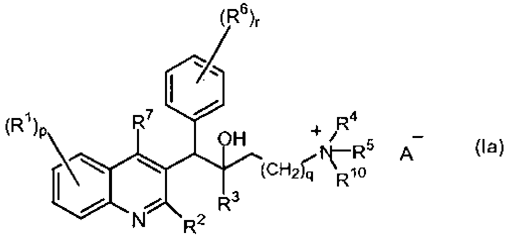 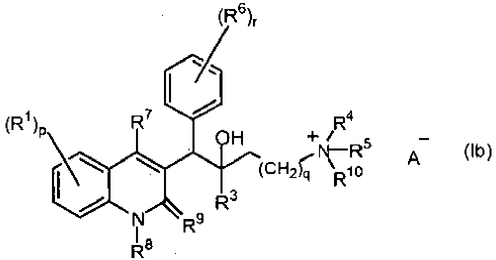 un N-oxyde de celui-ci, une forme tautomère de celui-ci ou une forme stéréochimiquement isomère de celui-ci dans lequel
A⁻ est un contre-ion pharmaceutiquement acceptable ;
R¹ est hydrogène, halogéno, halogénoalkyle, cyano, hydroxy, Ar, Het, alkyle, alkyloxy, alkylthio, alkyloxyalkyle, alkylthioalkyle, Ar-alkyle ou di(Ar)alkyle ;
p est un entier égal à 1, 2, 3 ou 4 ;
R² est hydrogène, hydroxy, mercapto, alkyloxy, alkyloxyalkyloxy, alkylthio, mono ou di(alkyl)amino ou
un radical de formule 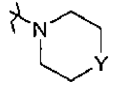 dans lequel Y est CH₂, O, S, NH ou N-alkyle ;
R³ est alkyle, Ar, Ar-alkyle, Het ou Het-alkyle ;
q est un entier égal à zéro, 1, 2, 3 ou 4 ;
R⁴ et R⁵ sont chacun indépendamment hydrogène, alkyle ou benzyle ;
R⁴ et R⁵ conjointement et comprenant le N auquel ils sont liés peuvent former un radical choisi dans le groupe de pyrrolidinyle, 2-pyrrolinyle, 3-pyrrolinyle, pyrrolyle, imidazolidinyle, pyrazolidinyle, 2-imidazolinyle, 2-pyrazolinyle, imidazolyle, pyrazolyle, triazolyle, pipéridinyle, pyridinyle, pipérazinyle, pyridazinyle, pyrimidinyle, pyrazinyle, triazinyle, morpholinyle et thiomorpholinyle, chacun desdits cycles peut facultativement être substitué par alkyle, halogéno, halogénoalkyle, hydroxy, alkyloxy, amino, mono- ou dialkylamino, alkylthio, alkyloxyalkyle, alkylthioalkyle, Ar-alkyle ou pyrimidinyle ;
R⁶ est hydrogène, halogéno, halogénoalkyle, hydroxy, Ar, alkyle, alkyloxy, alkylthio, alkyloxyalkyle, alkylthioalkyle, Ar-alkyle ou di(Ar)alkyle ; ou
deux radicaux R⁶ vicinaux peuvent former conjointement un radical bivalent de formule -CH=CH-CH=CH- ;
r est un entier égal à 1, 2, 3, 4 ou 5 ;
R⁷ est hydrogène, alkyle, Ar ou Het ;
R⁸ est hydrogène ou alkyle ;
R⁹ est oxo ; ou
R⁸ et R⁹ forment conjointement le radical -CH=CH-N= ;
R¹⁰ est alkyle, alkylcarbonyle, Ar, Ar-alkyle, Arcarbonyle, Het¹-alkyle ou Het¹-carbonyle ;
alkyle est un radical hydrocarboné linéaire ou ramifié ayant de 1 à 6 atomes de carbone ; ou est un radical hydrocarboné saturé cyclique ayant de 3 à 6 atomes de carbone ; ou est un radical hydrocarboné saturé cyclique ayant de 3 à 6 atomes de carbone lié à un radical hydrocarboné saturé linéaire ou ramifié ayant de 1 à 6 atomes de carbone ; chaque atome de carbone pouvant être facultativement substitué par hydroxy, alkyloxy ou oxo ;
Ar est un homocycle choisi dans le groupe de phényle, naphtyle, acénaphtyle, tétrahydronaphtyle, chaque homocycle étant facultativement substitué par 1, 2 ou 3 substituants, chaque substituant étant indépendamment choisi dans le groupe d'hydroxy, halogéno, cyano, nitro, amino, mono- ou dialkylamino, alkyle, halogénoalkyle, alkyloxy, halogénoalkyloxy, carboxyle, alkyloxycarbonyle, aminocarbonyle, morpholinyle et mono- ou dialkylaminocarbonyle ;
Het est un hétérocycle monocyclique choisi dans le groupe de N-phénoxypipéridinyle, pipéridinyle, pyrrolyle, pyrazolyle, imidazolyle, furanyle, thiényle, oxazolyle, isoxazolyle, thiazolyle, isothiazolyle, pyridinyle, pyrimidinyle, pyrazinyle et pyridazinyle ; ou un hétérocycle bicyclique choisi dans le groupe de quinoléinyle, quinoxalinyle, indolyle, benzimidazolyle, benzoxazolyle, benzisoxazolyle, benzothiazolyle, benzisothiazolyle, benzofuranyle, benzothiényle, 2,3-dihydrobenzo[1,4]dioxinyle et benzo[1,3]dioxolyle ; chaque hétérocycle monocyclique et bicyclique pouvant facultativement être substitué par 1, 2 ou 3 substituants,
chaque substituant étant indépendamment choisi dans le groupe d'halogéno, hydroxy, alkyle, alkyloxy, et Ar-carbonyle ;
Het¹ est un hétérocycle monocyclique choisi parmi furanyle ou thiényle ; ou un hétérocycle bicyclique choisi parmi benzofuranyle ou benzothiényle ; chaque hétérocycle monocyclique et bicyclique peut facultativement être substitué par 1, 2 ou 3 substituants, chaque substituant étant indépendamment choisi dans le groupe d'halogéno, alkyle et Ar ; halogéno est un substituant choisi dans le groupe de fluoro, chloro, bromo et iodo ; et
halogénoalkyle est un radical hydrocarboné linéaire ou ramifié, saturé ayant de 1 à 6 atomes de carbone ou un radical hydrocarboné cyclique saturé ayant de 3 à 6 atomes de carbone ou un radical hydrocarboné cyclique saturé ayant de 3 à 6 atomes de carbone lié à un radical hydrocarboné linéaire ou ramifié, saturé ayant de 1 à 6 atomes de carbone ; où un ou plusieurs atomes de carbone sont substitués par un ou plusieurs atomes d'halogéno.

2. Utilisation selon la revendication 1 dans laquelle le composé de formule (la) ou (Ib) est un composé ayant la formule suivante 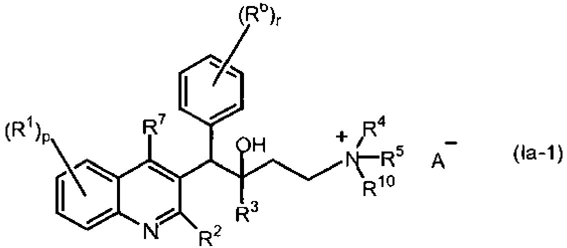 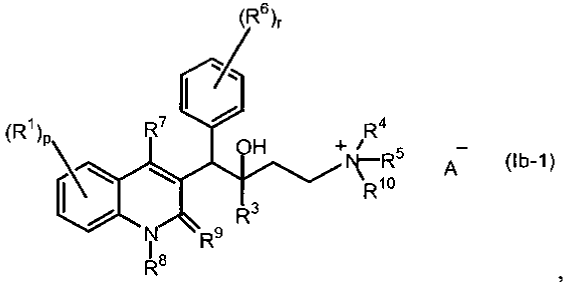 un N-oxyde de celui-ci, une forme tautomère de celui-ci ou une forme stéréochimiquement isomère de celui-ci.

3. Utilisation selon la revendication 1 dans laquelle le composé de formule (la) ou (Ib) est un composé ayant la formule suivante 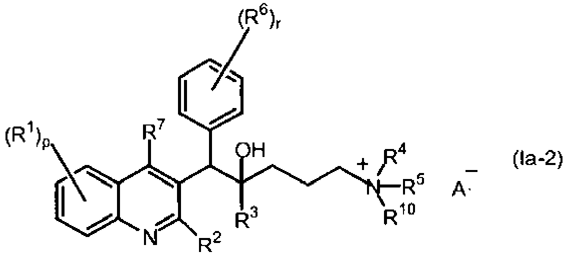 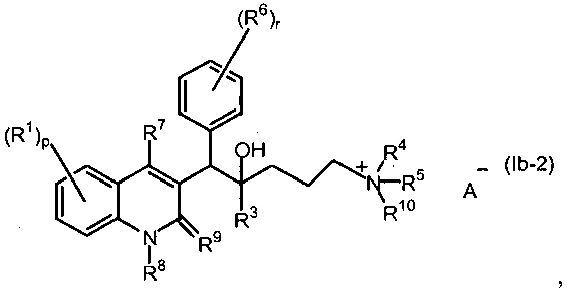 un N-oxyde de celui-ci, une forme tautomère de celui-ci ou une forme stéréochimiquement isomère de celui-ci.

4. Utilisation selon la revendication 1 dans laquelle le composé de formule (la) ou (Ib) est un composé ayant la formule suivante 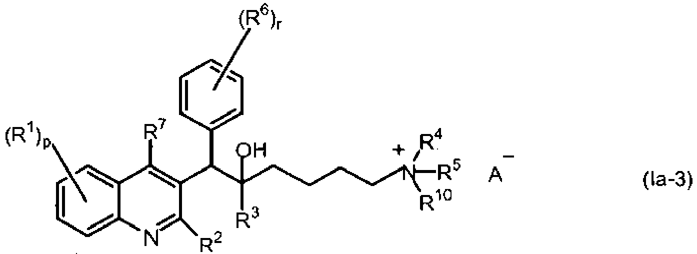 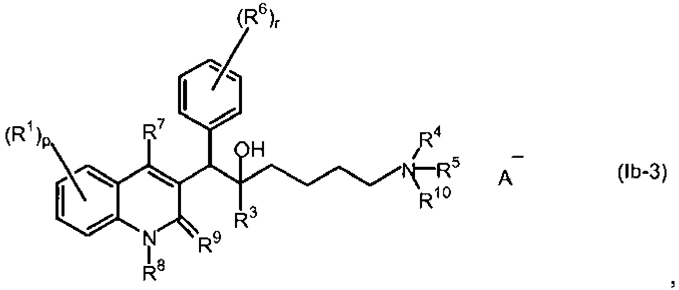 un N-oxyde de celui-ci, une forme tautomère de celui-ci ou une forme stéréochimiquement isomère de celui-ci.

5. Utilisation selon l'une quelconque des revendications précédentes dans laquelle R¹ est halogéno.

6. Utilisation selon l'une quelconque des revendications précédentes dans laquelle p est égal à 1.

7. Utilisation selon l'une quelconque des revendications précédentes dans laquelle R² est alkyloxy ou alkylthio.

8. Utilisation selon la revendication 7 dans laquelle R² est alkyloxy en C₁₋₄.

9. Utilisation selon l'une quelconque des revendications précédentes dans laquelle R³ est Het, Ar ou Ar-alkyle.

10. Utilisation selon la revendication 9 dans laquelle R³ est Ar ou Ar-alkyle.

11. Utilisation selon la revendication 9 dans laquelle R³ est thiényle, naphtyle, phényle, naphtyl-(alkyle en C₁₋₄) ou phényl- (alkyle en C₁₋₄).

12. Utilisation selon la revendication 11 dans laquelle R³ est naphtyle, phényle ou phényl-(alkyle en C₁₋₄).

13. Utilisation selon l'une quelconque des revendications précédentes dans laquelle R⁴ et R⁵ sont alkyle en C₁₋₄ ou R⁴ et R⁵ conjointement et comprenant le N auquel ils sont liés peuvent former un radical choisi parmi imidazolyle ou pipéridinyle.

14. Utilisation selon la revendication 13 dans laquelle R⁴ et R⁵ sont alkyle en C₁₋₄.

15. Utilisation selon l'une quelconque des revendications précédentes dans laquelle R⁶ est hydrogène ou halogéno.

16. Utilisation selon l'une quelconque des revendications précédentes dans laquelle r est égal à 1.

17. Utilisation selon l'une quelconque des revendications précédentes dans laquelle R⁷ est hydrogène.

18. Utilisation selon l'une quelconque des revendications précédentes dans laquelle R¹⁰ est alkyle.

19. Utilisation selon la revendication 18 dans laquelle R¹⁰ est alkyle en C₁₋₆.

20. Utilisation selon l'une quelconque des revendications précédentes dans laquelle A⁻ est iodure.

21. Utilisation selon l'une quelconque des revendications précédentes dans laquelle le composé est un composé selon la formule (la).

22. Utilisation d'un composé de formule (la) selon la revendication 1 dans laquelle R¹ est halogéno ; p = 1 ; R² est alkyloxy ou alkylthio ; R³ est naphtyle, phényle, phényléthyle ou thiényle ; q = 1, 2 ou 3 ; R⁴ et R⁵ sont alkyle en C₁₋₄ ou R⁴ et R⁵ conjointement et comprenant le N auquel ils sont liés peuvent former un radical choisi parmi imidazolyle ou pipéridinyle ; R⁶ est hydrogène ou halogéno ; r est égal à 1 ; R⁷ est hydrogène ; R¹⁰ est alkyle en C₁₋₆ ; A⁻ est iodure.

23. Utilisation selon la revendication 1 dans laquelle le composé est choisi parmi les composés suivants 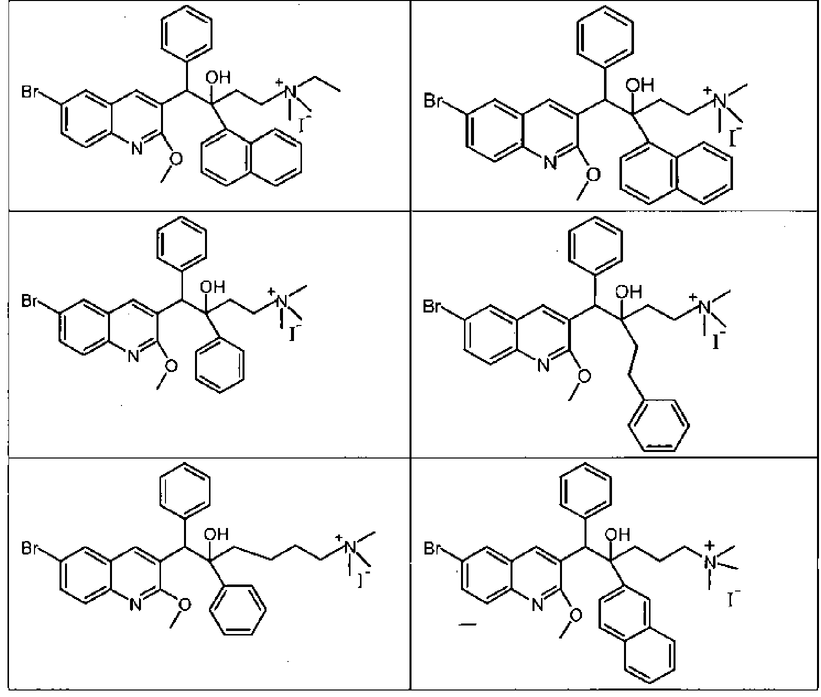 un N-oxyde de celui-ci ou une forme stéréochimiquement isomère de celui-ci.

24. Utilisation selon l'une quelconque des revendications précédentes dans laquelle l'infection bactérienne est une infection par une bactérie Gram positif.

25. Composé de formule (la) ou (Ib) 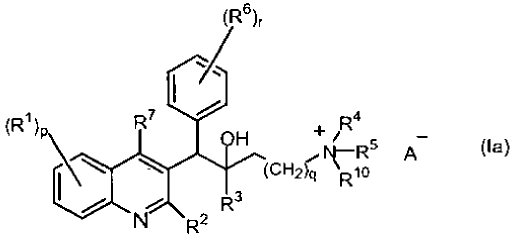 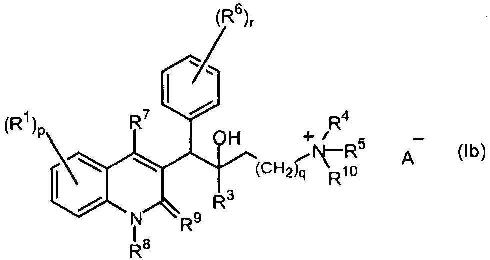 un N-oxyde de celui-ci, une forme tautomère de celui-ci ou une forme stéréochimiquement isomère de celui-ci dans lequel R¹, p, R², R³, q, R⁴, R⁵, R⁶, r, R⁷, R⁸, R⁹, R¹⁰ et A⁻ sont tels que définis dans la revendication 1 ;
à condition que, lorsque R¹⁰ est alkyle ou benzyle, R⁴ et R⁵ soient autres qu'hydrogène ; et à condition que le composé soit autre que 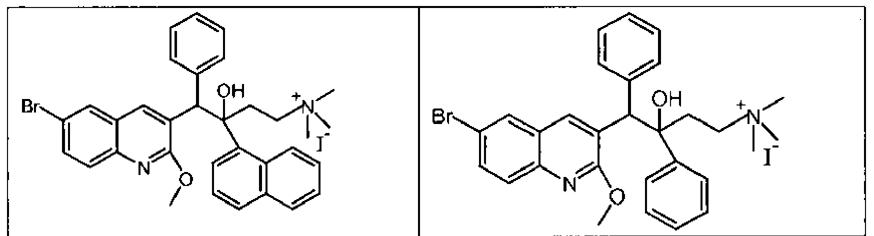 un N-oxyde de celui-ci, une forme tautomère de celui-ci ou une forme stéréochimiquement isomère de celui-ci.

26. Composé selon la revendication 25, le composé étant choisi parmi : 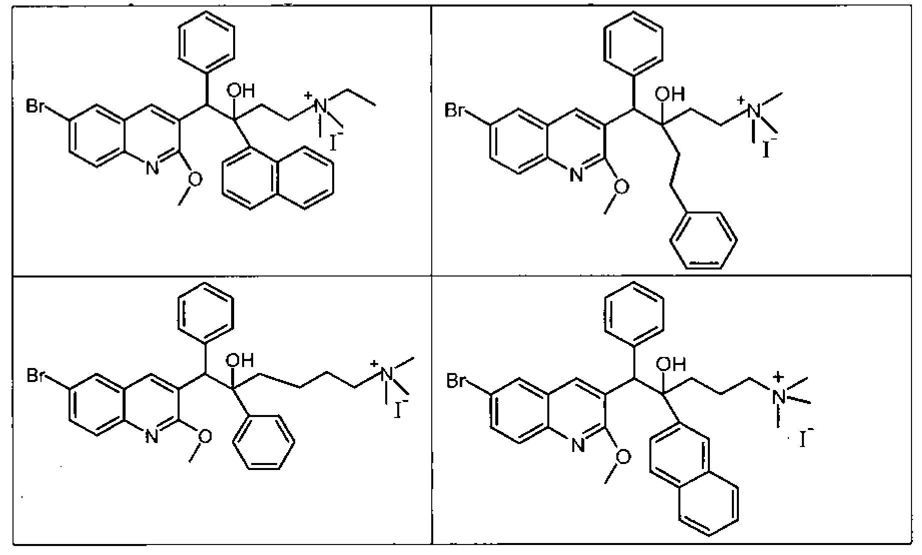 un N-oxyde de celui-ci ou une forme stéréochimiquement isomère de celui-ci.

27. Composé selon la revendication 26, le composé étant choisi parmi :
| | |
|---|---|
| 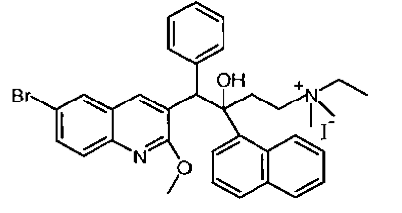 | 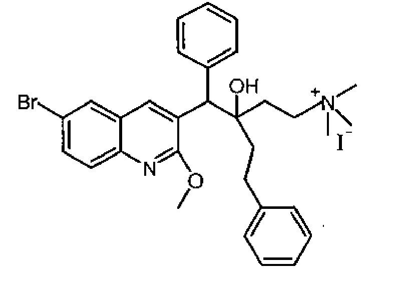 |
| (diastéréoisomère B) | (diastéréoisomère A) |
| 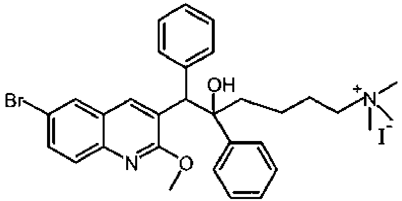 | 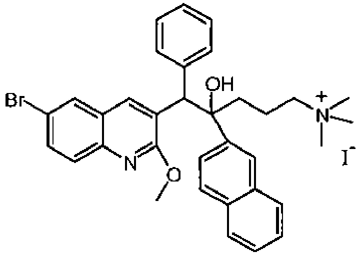 |
| (diastéréoisomère A) | (diastéréoisomère B) |
ou une forme stéréochimiquement isomère de celui-ci, où la forme stéréochimiquement isomère qui a été initialement isolée est appelée « A » et la deuxième « B ».

28. Combinaison de (a) un composé de formule (la) ou (Ib) tel que défini dans l'une quelconque des revendications précédentes, et (b) un ou plusieurs autre agents antibactériens à condition que les un ou plusieurs autres agents antibactériens soient autres que des agents antimycobactériens, la combinaison étant pour utilisation dans le traitement d'une infection bactérienne causée par des staphylocoques, des entérocoques ou des streptocoques.

29. Composition pharmaceutique comprenant un véhicule pharmaceutiquement acceptable et, en tant que substance active, une quantité thérapeutiquement efficace de (a) un composé de formule (la) ou (Ib) tel que défini dans l'une quelconque des revendications 1 à 27, et (b) un ou plusieurs autres agents antibactériens à condition que les un ou plusieurs autres agents antibactériens soient autres que des agents antimycobactériens, la composition étant pour utilisation dans le traitement d'une infection bactérienne causée par des staphylocoques, des entérocoques ou des streptocoques.

30. Composé tel que défini dans l'une quelconque des revendications 1 à 27, pour utilisation dans le traitement d'une infection bactérienne causée par des staphylocoques, des entérocoques ou des streptocoques.

31. Produit contenant (a) un composé de formule (la) ou (Ib) tel que défini dans l'une quelconque des revendications 1 à 27, et (b) un ou plusieurs autre agents antibactériens à condition que les un ou plusieurs autres agents antibactériens soient autres que des agents antimycobactériens, sous la forme d'une préparation combinée pour utilisation simultanée, séparée ou séquentielle dans le traitement d'une infection bactérienne causée par des staphylocoques, des entérocoques ou des streptocoques.

32. Utilisation selon l'une quelconque des revendications 1 à 24, combinaison selon la revendication 28, composition selon la revendication 29, composé selon la revendication 30 ou produit selon la revendication 31, l'infection bactérienne étant une infection par *Staphylococcus aureus* résistant à la méthicilline (SARM), des staphylocoques négatifs pour la coagulase résistants à la méthicilline (MRCNS), *Streptococcus pneumoniae* résistant à la pénicilline ou *Enterococcus faecium* à multirésistant.

33. Utilisation, combinaison, composition, composé ou produit selon la revendication 32, l'infection bactérienne étant une infection par *Staphylococcus aureus* ou *Streptococcus pneumoniae.*

34. Utilisation, combinaison, composition, composé ou produit selon la revendication 33, l'infection bactérienne étant une infection par *Staphylococcus aureus* (SARM).
